# EUROPEAN PATENT APPLICATION

(11) **EP 4 050 022 A1**
(43) Date of publication of application: **31.08.2022**
(21) Application number: 20901728.4
(22) Date of filing: 18.12.2020
(51) Int. Cl.: C07K 14/245, C12N 15/77, C12P 13/06, C12P 13/12

(54) **MUTANT OF INNER MEMBRANE PROTEIN, AND METHOD FOR PRODUCING TARGET PRODUCT BY USING SAME**

(30) Priority: 20.12.2019 KR 20190172055
(71) Applicant: CJ Cheiljedang Corporation, Seoul 04560 (KR)
(72) Inventor: KIM, Seo Yun, Seoul 04560 (KR); CHO, Seung Hyun, Seoul 04560 (KR); LEE, Jae Min, Seoul 04560 (KR); BAEK, Min Ji, Seoul 04560 (KR)
(74) Representative: Regimbeau
(86) International application number: PCT/KR2020/018713
(87) International publication number: WO 2021/125896

(57) **Abstract**

The present disclosure relates to a variant of YjeH, which is an inner membrane protein, a microorganism including the same, and a method for producing a target product using the same.

<110>    CJ CheilJedang Corporation

<120>    A modified inner membrane protein and methods for producing
         purpose product using them

<130>    OPA20205

<150>    KR 10-2019-0172055
<151>    2019-12-20

<160>    37

<170>    KoPatentIn 3.0

<210>    1
<211>    418
<212>    PRT
<213>    Unknown

<220>
<223>    Escherichia coli YjeH


<400>    1
   Met Ser Gly Leu Lys Gln Glu Leu Gly Leu Ala Gln Gly Ile Gly Leu
     1               5                  10                  15
   Leu Ser Thr Ser Leu Leu Gly Thr Gly Val Phe Ala Val Pro Ala Leu
                20                  25                  30
   Ala Ala Leu Val Ala Gly Asn Asn Ser Leu Trp Ala Trp Pro Val Leu
            35                  40                  45
   Ile Ile Leu Val Phe Pro Ile Ala Ile Val Phe Ala Ile Leu Gly Arg
        50                  55                  60
   His Tyr Pro Ser Ala Gly Gly Val Ala His Phe Val Gly Met Ala Phe
    65                  70                  75                  80
   Gly Ser Arg Leu Glu Arg Val Thr Gly Trp Leu Phe Leu Ser Val Ile
                    85                  90                  95
   Pro Val Gly Leu Pro Ala Ala Leu Gln Ile Ala Ala Gly Phe Gly Gln
               100                 105                 110
   Ala Met Phe Gly Trp His Ser Trp Gln Leu Leu Leu Ala Glu Leu Gly
           115                 120                 125
   Thr Leu Ala Leu Val Trp Tyr Ile Gly Thr Arg Gly Ala Ser Ser Ser
       130                 135                 140
   Ala Asn Leu Gln Thr Val Ile Ala Gly Leu Ile Val Ala Leu Ile Val
   145                 150                 155                 160
   Ala Ile Trp Trp Ala Gly Asp Ile Lys Pro Ala Asn Ile Pro Phe Pro
                   165                 170                 175
   Ala Pro Gly Asn Ile Glu Leu Thr Gly Leu Phe Ala Ala Leu Ser Val
               180                 185                 190
   Met Phe Trp Cys Phe Val Gly Leu Glu Ala Phe Ala His Leu Ala Ser
           195                 200                 205
   Glu Phe Lys Asn Pro Glu Arg Asp Phe Pro Arg Ala Leu Met Ile Gly
       210                 215                 220
   Leu Leu Leu Ala Gly Leu Val Tyr Trp Gly Cys Thr Val Val Val Leu
   225                 230                 235                 240
   His Phe Asp Ala Tyr Gly Glu Lys Met Ala Ala Ala Ala Ser Leu Pro
                   245                 250                 255
   Lys Ile Val Val Gln Leu Phe Gly Val Gly Ala Leu Trp Ile Ala Cys
               260                 265                 270
   Val Ile Gly Tyr Leu Ala Cys Phe Ala Ser Leu Asn Ile Tyr Ile Gln
           275                 280                 285
   Ser Phe Ala Arg Leu Val Trp Ser Gln Ala Gln His Asn Pro Asp His
       290                 295                 300
   Tyr Leu Ala Arg Leu Ser Ser Arg His Ile Pro Asn Asn Ala Leu Asn
   305                 310                 315                 320
   Ala Val Leu Gly Cys Cys Val Val Ser Thr Leu Val Ile His Ala Leu
                   325                 330                 335
   Glu Ile Asn Leu Asp Ala Leu Ile Ile Tyr Ala Asn Gly Ile Phe Ile
               340                 345                 350
   Met Ile Tyr Leu Leu Cys Met Leu Ala Gly Cys Lys Leu Leu Gln Gly
           355                 360                 365
   Arg Tyr Arg Leu Leu Ala Val Val Gly Gly Leu Leu Cys Val Leu Leu
       370                 375                 380
   Leu Ala Met Val Gly Trp Lys Ser Leu Tyr Ala Leu Ile Met Leu Ala
   385                 390                 395                 400
   Gly Leu Trp Leu Leu Leu Pro Lys Arg Lys Thr Pro Glu Asn Gly Ile
                   405                 410                 415
   Thr Thr

<210>    2
<211>    1257
<212>    DNA
<213>    Unknown

<220>
<223>    Escherichia coli yjeH

<400>    2
atgagtggac tcaaacaaga actggggctg gcccagggca ttggcctgct atcgacgtca      60

ttattaggca ctggcgtgtt tgccgttcct gcgttagctg cgctggtagc gggcaataac     120

agcctgtggg cgtggcccgt tttgattatc ttagtgttcc cgattgcgat tgtgtttgcg     180

attctgggtc gccactatcc cagcgcaggc ggcgtcgcgc acttcgtcgg tatggcgttt     240

ggttcgcggc ttgagcgagt caccggctgg ctgtttttat cggtcattcc cgtgggtttg     300

cctgccgcac tacaaattgc cgccgggttc ggccaggcga tgtttggctg gcatagctgg 360

caactgttgt tggcagaact cggtacgctg gcgctggtgt ggtatatcgg tactcgcggt 420

gccagttcca gtgctaatct acaaaccgtt attgccggac ttatcgtcgc gctgattgtc 480

gctatctggt gggcgggcga tatcaaacct gcgaatatcc cctttccggc acctggtaat 540

atcgaactta ccgggttatt tgctgcgtta tcagtgatgt tctggtgttt tgtcggtctg 600

gaggcatttg cccatctcgc ctcggaattt aaaaatccag agcgtgattt tcctcgtgct 660

ttgatgattg gtctgctgct ggcaggatta gtctactggg gctgtacggt agtcgtctta 720

cacttcgacg cctatggtga aaaaatggcg gcggcagcat cgcttccaaa aattgtagtg 780

cagttgttcg gtgtaggagc gttatggatt gcctgcgtga ttggctatct ggcctgcttt 840

gccagtctca acatttatat acagagcttc gcccgcctgg tctggtcgca ggcgcaacat 900

aatcctgacc actacctggc acgcctctct tctcgccata tcccgaataa tgccctcaat 960

gcggtgctcg gctgctgtgt ggtgagcact ttggtgattc atgctttaga gatcaatctg 1020

gacgctctta ttatttatgc caatggcatc tttattatga tttatctgtt atgcatgctg 1080

gcaggctgta aattattgca aggacgttat cgactactgg cggtggttgg cgggctgtta 1140

tgcgttctgt tactggcaat ggtcggctgg aaaagtctct atgcgctgat catgctggcg 1200

gggttatggc tgttgctgcc aaaacgaaaa acgccggaaa atggcataac cacataa 1257

<210> 3
<211> 267
<212> DNA
<213> Artificial Sequence

<220>
<223> Tn2335

<400> 3
gtggcctaca ccttcgacca cgtcgtcgcc tggcgctggt gcaccaaaga agacgcctac 60

aactacaccc acctcttcga tcaactccaa ccacccttaa tcgtgaccac cgacggacaa 120

aaaaggcgca ctcaaagcca tcaccacgac ctggccgaca acgaaaatcc aacgctgcct 180

cgtccacgtc aaacgcaacg tccaaaaaca cgtcacccta agacccgtgc tgagctcgcc 240

gaaaagcact ccggggtctc tccttga 267

<210> 4
<211> 39
<212> DNA
<213> Artificial Sequence

<220>
<223> Tn_5 F

<400>    4
tgaattcgag ctcggtaccc caccgacgcg catctgcct                              39


<210>    5
<211>    50
<212>    DNA
<213>    Artificial Sequence

<220>
<223>    Tn_5 R


<400>    5
ggtgtggtga ctttcagcag ttcccggggg ggaggaggca tgtggtgttg                  50


<210>    6
<211>    50
<212>    DNA
<213>    Artificial Sequence

<220>
<223>    Tn_3 F


<400>    6
caacaccaca tgcctcctcc cccccgggaa ctgctgaaag tcaccacacc                  50


<210>    7
<211>    42
<212>    DNA
<213>    Artificial Sequence

<220>
<223>    Tn_3 R


<400>    7
gtcgactcta gaggatcccc ctcccaaacc attgaggaat gg                          42


<210>    8
<211>    318
<212>    DNA
<213>    Artificial Sequence

<220>
<223>    CJ7


<400>    8
agaaacatcc cagcgctact aatagggagc gttgaccttc cttccacgga ccggtaatcg       60

gagtgcctaa aaccgcatgc ggcttaggct ccaagatagg ttctgcgcgg ccgggtaatg      120

catcttcttt agcaacaagt tgaggggtag gtgcaaataa gaacgacata gaaatcgtct      180

cctttctgtt tttaatcaac atacaccacc acctaaaaat tccccgacca gcaagttcac      240

agtattcggg cacaatatcg ttgccaaaat attgtttcgg aatatcatgg gatacgtacc      300

caacgaaagg aaacactc                                                    318

<210>    9
<211>    39
<212>    DNA
<213>    Artificial Sequence

<220>
<223>    CJ7_yjeH F

<400>    9
acaccacatg cctcctcccc agaaacatcc cagcgctac                             39

<210>    10
<211>    43
<212>    DNA
<213>    Artificial Sequence

<220>
<223>    CJ7_yjeH R

<400>    10
agttcttgtt tgagtccact catagtgttt cctttcgttg ggt                        43

<210>    11
<211>    44
<212>    DNA
<213>    Artificial Sequence

<220>
<223>    yjeH F

<400>    11
acccaacgaa aggaaacact atgagtggac tcaaacaaga actg                       44

<210>    12
<211>    44
<212>    DNA
<213>    Artificial Sequence

<220>
<223>    yjeH R

<400>    12
gactttcagc agttcccggg ttatgtggtt atgccatttt ccgg                       44

<210>    13
<211>    23
<212>    DNA
<213>    Artificial Sequence

<220>
<223>    F92N F

<400>    13
cggctggctg aatttatcgg tca                                              23


<210>    14
<211>    23
<212>    DNA
<213>    Artificial Sequence

<220>
<223>    F92N R


<400>    14
tgaccgataa attcagccag ccg                                              23


<210>    15
<211>    23
<212>    DNA
<213>    Artificial Sequence

<220>
<223>    F351L F


<400>    15
caatggcatc cttattatga ttt                                              23


<210>    16
<211>    23
<212>    DNA
<213>    Artificial Sequence

<220>
<223>    F351L R


<400>    16
aaatcataat aaggatgcca ttg                                              23


<210>    17
<211>    1161
<212>    DNA
<213>    Unknown

<220>
<223>    Corynebacterium glutamicum metB


<400>    17
ttgtcttttg acccaaacac ccagggtttc tccactgcat cgattcacgc tgggtatgag      60

ccagacgact actacggttc gattaacacc ccaatctatg cctccaccac cttcgcgcag     120

aacgctccaa acgaactgcg caaaggctac gagtacaccc gtgtgggcaa ccccaccatc     180

gtggcattag agcagaccgt cgcagcactc gaaggcgcaa agtatggccg cgcattctcc     240

gtcctcggca acgatgctta cggcggaacc taccgcctga tcgacaccgt attcaccgca 360

tggggcgtcg aatacaccgt tgttgatacc tccgtcgtgg aagaggtcaa ggcagcgatc 420

aaggacaaca ccaagctgat ctgggtggaa accccaacca acccagcact tggcatcacc 480

gacatcgaag cagtagcaaa gctcaccgaa ggcaccaacg ccaagctggt tgttgacaac 540

accttcgcat ccccatacct gcagcagcca ctaaaactcg gcgcacacgc agtcctgcac 600

tccaccacca agtacatcgg aggacactcc gacgttgttg gcggccttgt ggttaccaac 660

gaccaggaaa tggacgaaga actgctgttc atgcagggcg gcatcggacc gatcccatca 720

gttttcgatg catacctgac cgcccgtggc ctcaagaccc ttgcagtgcg catggatcgc 780

cactgcgaca acgcagaaaa gatcgcggaa ttcctggact cccgcccaga ggtctccacc 840

gtgctctacc caggtctgaa gaaccaccca ggccacgaag tcgcagcgaa gcagatgaag 900

cgcttcggcg gcatgatctc cgtccgtttc gcaggcggcg aagaagcagc taagaagttc 960

tgtacctcca ccaaactgat ctgtctggcc gagtccctcg gtggcgtgga atccctcctg 1020

gagcacccag caaccatgac ccaccagtca gctgccggct ctcagctcga ggttccccgc 1080

gacctcgtgc gcatctccat tggtattgaa gacattgaag acctgctcgc agatgtcgag 1140

caggccctca ataaccttta g 1161

<210> 18
<211> 24
<212> DNA
<213> Artificial Sequence

<220>
<223> metB_N_del F

<400> 18
tctagacgcc cgcatactgg cttc 24

<210> 19
<211> 37
<212> DNA
<213> Artificial Sequence

<220>
<223> metB_N_del R

<400> 19
cccatccact aaacttaaac agatgtgatc gcccggc 37

<210> 20
<211> 38
<212> DNA
<213> Artificial Sequence

<220>
<223>    metB_C_del F


<400>    20
tgtttaagtt tagtggatgg ggaagaacca cccaggcc                              38


<210>    21
<211>    23
<212>    DNA
<213>    Artificial Sequence

<220>
<223>    metB_C_del R


<400>    21
gtcgaccaat cgtccagagg gcg                                              23


<210>    22
<211>    1314
<212>    DNA
<213>    Artificial Sequence

<220>
<223>    Corynebacterium glutamicum metY


<400>    22
atgccaaagt acgacaattc caatgctgac cagtggggct ttgaaacccg ctccattcac      60

gcaggccagt cagtagacgc acagaccagc gcacgaaacc ttccgatcta ccaatccacc     120

gctttcgtgt tcgactccgc tgagcacgcc aagcagcgtt tcgcacttga ggatctaggc     180

cctgtttact cccgcctcac caacccaacc gttgaggctt tggaaaaccg catcgcttcc     240

ctcgaaggtg gcgtccacgc tgtagcgttc tcctccggac aggccgcaac caccaacgcc     300

attttgaacc tggcaggagc gggcgaccac atcgtcacct ccccacgcct ctacggtggc     360

accgagactc tattccttat cactcttaac cgcctgggta tcgatgtttc cttcgtggaa     420

aaccccgacg accctgagtc ctggcaggca gccgttcagc caaacaccaa agcattcttc     480

ggcgagactt tcgccaaccc acaggcagac gtcctggata ttcctgcggt ggctgaagtt     540

gcgcaccgca acagcgttcc actgatcatc gacaacacca tcgctaccgc agcgctcgtg     600

cgcccgctcg agctcggcgc agacgttgtc gtcgcttccc tcaccaagtt ctacaccggc     660

aacggctccg gactgggcgg cgtgcttatc gacggcggaa agttcgattg gactgtcgaa     720

aaggatggaa agccagtatt cccctacttc gtcactccag atgctgctta ccacggattg     780

aagtacgcag accttggtgc accagccttc ggcctcaagg ttcgcgttgg ccttctacgc     840

gacaccggct ccaccctctc cgcattcaac gcatgggctg cagtccaggg catcgacacc     900

ctttccctgc gcctggagcg ccacaacgaa aacgccatca aggttgcaga attcctcaac     960

aaccacgaga aggtggaaaa ggttaacttc gcaggcctga aggattcccc ttggtacgca 1020

accaaggaaa agcttggcct gaagtacacc ggctccgttc tcaccttcga gatcaagggc 1080

ggcaaggatg aggcttgggc atttatcgac gccctgaagc tacactccaa ccttgcaaac 1140

atcggcgatg ttcgctccct cgttgttcac ccagcaacca ccacccattc acagtccgac 1200

gaagctggcc tggcacgcgc gggcgttacc cagtccaccg tccgcctgtc cgttggcatc 1260

gagaccattg atgatatcat cgctgacctc gaaggcggct ttgctgcaat ctag 1314

<210> 23
<211> 24
<212> DNA
<213> Artificial Sequence

<220>
<223> metY_N_del F

<400> 23
tctagaccat cctgcaccat ttag 24

<210> 24
<211> 37
<212> DNA
<213> Artificial Sequence

<220>
<223> metY_N_del R

<400> 24
cccatccact aaacttaaac acgctcctgc caggttc 37

<210> 25
<211> 39
<212> DNA
<213> Artificial Sequence

<220>
<223> metY_C_del F

<400> 25
tgtttaagtt tagtggatgg gcttggtacg caaccaagg 39

<210> 26
<211> 23
<212> DNA
<213> Artificial Sequence

<220>
<223> metY_C_del R

<400> 26
gtcgacgatt gctccggctt cgg 23

<210>     27
<211>     1266
<212>     DNA
<213>     Unknown

<220>
<223>     Corynebacterium glutamicum lysC


<400>     27
gtggccctgg tcgtacagaa atatggcggt tcctcgcttg agagtgcgga acgcattaga          60

aacgtcgctg aacggatcgt tgccaccaag aaggctggaa atgatgtcgt ggttgtctgc         120

tccgcaatgg gagacaccac ggatgaactt ctagaacttg cagcggcagt gaatcccgtt         180

ccgccagctc gtgaaatgga tatgctcctg actgctggtg agcgtatttc taacgctctc         240

gtcgccatgg ctattgagtc ccttggcgca gaagcccaat ctttcacggg ctctcaggct         300

ggtgtgctca ccaccgagcg ccacggaaac gcacgcattg ttgatgtcac tccaggtcgt         360

gtgcgtgaag cactcgatga gggcaagatc tgcattgttg ctggtttcca gggtgttaat         420

aaagaaaccc gcgatgtcac cacgttgggt cgtggtggtt ctgacaccac tgcagttgcg         480

ttggcagctg ctttgaacgc tgatgtgtgt gagatttact cggacgttga cggtgtgtat         540

accgctgacc cgcgcatcgt tcctaatgca cagaagctgg aaaagctcag cttcgaagaa         600

atgctggaac ttgctgctgt tggctccaag attttggtgc tgcgcagtgt tgaatacgct         660

cgtgcattca atgtgccact tcgcgtacgc tcgtcttata gtaatgatcc cggcactttg         720

attgccggct ctatggagga tattcctgtg gaagaagcag tccttaccgg tgtcgcaacc         780

gacaagtccg aagccaaagt aaccgttctg ggtatttccg ataagccagg cgaggctgcg         840

aaggttttcc gtgcgttggc tgatgcagaa atcaacattg acatggttct gcagaacgtc         900

tcttctgtag aagacggcac caccgacatc accttcacct gccctcgttc cgacggccgc         960

cgcgcgatgg agatcttgaa gaagcttcag gttcagggca actggaccaa tgtgctttac        1020

gacgaccagg tcggcaaagt ctccctcgtg ggtgctggca tgaagtctca cccaggtgtt        1080

accgcagagt tcatggaagc tctgcgcgat gtcaacgtga acatcgaatt gatttccacc        1140

tctgagattc gtatttccgt gctgatccgt gaagatgatc tggatgctgc tgcacgtgca        1200

ttgcatgagc agttccagct gggcggcgaa gacgaagccg tcgtttatgc aggcaccgga        1260

cgctaa                                                                    1266


<210>     28
<211>     29
<212>     DNA
<213>     Artificial Sequence

<220>

<223>    lysC_L377K_5 F

<400>    28
tcctctagag ctgcgcagtg ttgaatacg                                          29

<210>    29
<211>    23
<212>    DNA
<213>    Artificial Sequence

<220>
<223>    lysC_L377K_5 R

<400>    29
aggtggaaat cttttcgatg ttc                                                23

<210>    30
<211>    23
<212>    DNA
<213>    Artificial Sequence

<220>
<223>    lysC_L377K_3 F

<400>    30
gaacatcgaa aagatttcca cct                                                23

<210>    31
<211>    29
<212>    DNA
<213>    Artificial Sequence

<220>
<223>    lysC_L377K_3 R

<400>    31
gactctagag ttcacctcag agacgatta                                          29

<210>    32
<211>    1134
<212>    DNA
<213>    Artificial Sequence

<220>
<223>    Corynebacterium glutamicum  metX

<400>    32
atgcccaccc tcgcgccttc aggtcaactt gaaatccaag cgatcggtga tgtctccacc        60

gaagccggag caatcattac aaacgctgaa atcgcctatc accgctgggg tgaataccgc       120

gtagataaag aaggacgcag caatgtcgtt ctcatcgaac acgccctcac tggagattcc       180

aacgcagccg attggtgggc tgacttgctc ggtcccggca aagccatcaa cactgatatt       240

tactgcgtga tctgtaccaa cgtcatcggt ggttgcaacg gttccaccgg acctggctcc 300

atgcatccag atggaaattt ctggggtaat cgcttccccg ccacgtccat tcgtgatcag 360

gtaaacgccg aaaaacaatt cctcgacgca ctcggcatca ccacggtcgc cgcagtactt 420

ggtggttcca tgggtggtgc ccgcacccta gagtgggccg caatgtaccc agaaactgtt 480

ggcgcagctg ctgttcttgc agtttctgca cgcgccagcg cctggcaaat cggcattcaa 540

tccgcccaaa ttaaggcgat tgaaaacgac caccactggc acgaaggcaa ctactacgaa 600

tccggctgca acccagccac cggactcggc gccgcccgac gcatcgccca cctcacctac 660

cgtggcgaac tagaaatcga cgaacgcttc ggcaccaaag cccaaaagaa cgaaaaccca 720

ctcggtccct accgcaagcc cgaccagcgc ttcgccgtgg aatcctactt ggactaccaa 780

gcagacaagc tagtacagcg tttcgacgcc ggctcctacg tcttgctcac cgacgccctc 840

aaccgccacg acattggtcg cgaccgcgga ggcctcaaca aggcactcga atccatcaaa 900

gttccagtcc ttgtcgcagg cgtagatacc gatattttgt acccctacca ccagcaagaa 960

cacctctcca gaaacctggg aaatctactg gcaatggcaa aaatcgtatc ccctgtcggc 1020

cacgatgctt tcctcaccga aagccgccaa atggatcgca tcgtgaggaa cttcttcagc 1080

ctcatctccc cagacgaaga caacccttcg acctacatcg agttctacat ctaa 1134

<210> 33
<211> 28
<212> DNA
<213> Artificial Sequence

<220>
<223> metX F

<400> 33
ggatcccctc gttgttcacc cagcaacc 28

<210> 34
<211> 30
<212> DNA
<213> Artificial Sequence

<220>
<223> metX R

<400> 34
ggatcccaaa gtcacaacta cttatgttag 30

<210> 35
<211> 418
<212> PRT
<213> Artificial Sequence

<220>
<223>    YjeH F92N


<400>    35
Met Ser Gly Leu Lys Gln Glu Leu Gly Leu Ala Gln Gly Ile Gly Leu
1               5                   10                  15

Leu Ser Thr Ser Leu Leu Gly Thr Gly Val Phe Ala Val Pro Ala Leu
            20                  25                  30

Ala Ala Leu Val Ala Gly Asn Asn Ser Leu Trp Ala Trp Pro Val Leu
        35                  40                  45

Ile Ile Leu Val Phe Pro Ile Ala Ile Val Phe Ala Ile Leu Gly Arg
    50                  55                  60

His Tyr Pro Ser Ala Gly Gly Val Ala His Phe Val Gly Met Ala Phe
65                  70                  75                  80

Gly Ser Arg Leu Glu Arg Val Thr Gly Trp Leu Asn Leu Ser Val Ile
                85                  90                  95

Pro Val Gly Leu Pro Ala Ala Leu Gln Ile Ala Ala Gly Phe Gly Gln
            100                 105                 110

Ala Met Phe Gly Trp His Ser Trp Gln Leu Leu Leu Ala Glu Leu Gly
        115                 120                 125

Thr Leu Ala Leu Val Trp Tyr Ile Gly Thr Arg Gly Ala Ser Ser Ser
    130                 135                 140

Ala Asn Leu Gln Thr Val Ile Ala Gly Leu Ile Val Ala Leu Ile Val
145                 150                 155                 160

Ala Ile Trp Trp Ala Gly Asp Ile Lys Pro Ala Asn Ile Pro Phe Pro
                165                 170                 175

Ala Pro Gly Asn Ile Glu Leu Thr Gly Leu Phe Ala Ala Leu Ser Val
            180                 185                 190

Met Phe Trp Cys Phe Val Gly Leu Glu Ala Phe Ala His Leu Ala Ser
        195                 200                 205

Glu Phe Lys Asn Pro Glu Arg Asp Phe Pro Arg Ala Leu Met Ile Gly
    210                 215                 220

Leu Leu Leu Ala Gly Leu Val Tyr Trp Gly Cys Thr Val Val Val Leu
225                 230                 235                 240

His Phe Asp Ala Tyr Gly Glu Lys Met Ala Ala Ala Ala Ser Leu Pro
                245                 250                 255

Lys Ile Val Val Gln Leu Phe Gly Val Gly Ala Leu Trp Ile Ala Cys
            260                 265                 270

Val Ile Gly Tyr Leu Ala Cys Phe Ala Ser Leu Asn Ile Tyr Ile Gln
        275                 280                 285

Ser Phe Ala Arg Leu Val Trp Ser Gln Ala Gln His Asn Pro Asp His
    290                 295                 300

Tyr Leu Ala Arg Leu Ser Ser Arg His Ile Pro Asn Asn Ala Leu Asn

305                 310                 315                 320

Ala Val Leu Gly Cys Cys Val Val Ser Thr Leu Val Ile His Ala Leu
                325                 330                 335

Glu Ile Asn Leu Asp Ala Leu Ile Ile Tyr Ala Asn Gly Ile Phe Ile
            340                 345                 350

Met Ile Tyr Leu Leu Cys Met Leu Ala Gly Cys Lys Leu Leu Gln Gly
        355                 360                 365

Arg Tyr Arg Leu Leu Ala Val Val Gly Gly Leu Leu Cys Val Leu Leu
    370                 375                 380

Leu Ala Met Val Gly Trp Lys Ser Leu Tyr Ala Leu Ile Met Leu Ala
385                 390                 395                 400

Gly Leu Trp Leu Leu Leu Pro Lys Arg Lys Thr Pro Glu Asn Gly Ile
                405                 410                 415

Thr Thr

<210>    36
<211>    418
<212>    PRT
<213>    Artificial Sequence

<220>
<223>    YjeH F351L

<400>    36
Met Ser Gly Leu Lys Gln Glu Leu Gly Leu Ala Gln Gly Ile Gly Leu
1               5                   10                  15

Leu Ser Thr Ser Leu Leu Gly Thr Gly Val Phe Ala Val Pro Ala Leu
            20                  25                  30

Ala Ala Leu Val Ala Gly Asn Asn Ser Leu Trp Ala Trp Pro Val Leu
        35                  40                  45

Ile Ile Leu Val Phe Pro Ile Ala Ile Val Phe Ala Ile Leu Gly Arg
    50                  55                  60

His Tyr Pro Ser Ala Gly Gly Val Ala His Phe Val Gly Met Ala Phe
65                  70                  75                  80

Gly Ser Arg Leu Glu Arg Val Thr Gly Trp Leu Phe Leu Ser Val Ile
                85                  90                  95

Pro Val Gly Leu Pro Ala Ala Leu Gln Ile Ala Ala Gly Phe Gly Gln
            100                 105                 110

Ala Met Phe Gly Trp His Ser Trp Gln Leu Leu Leu Ala Glu Leu Gly
        115                 120                 125

Thr Leu Ala Leu Val Trp Tyr Ile Gly Thr Arg Gly Ala Ser Ser Ser
    130                 135                 140

Ala Asn Leu Gln Thr Val Ile Ala Gly Leu Ile Val Ala Leu Ile Val
145                 150                 155                 160

Ala Ile Trp Trp Ala Gly Asp Ile Lys Pro Ala Asn Ile Pro Phe Pro
                165                 170                 175

Ala Pro Gly Asn Ile Glu Leu Thr Gly Leu Phe Ala Ala Leu Ser Val
            180                 185                 190

Met Phe Trp Cys Phe Val Gly Leu Glu Ala Phe Ala His Leu Ala Ser
        195                 200                 205

Glu Phe Lys Asn Pro Glu Arg Asp Phe Pro Arg Ala Leu Met Ile Gly
    210                 215                 220

Leu Leu Leu Ala Gly Leu Val Tyr Trp Gly Cys Thr Val Val Val Leu
225                 230                 235                 240

His Phe Asp Ala Tyr Gly Glu Lys Met Ala Ala Ala Ala Ser Leu Pro
                245                 250                 255

Lys Ile Val Val Gln Leu Phe Gly Val Gly Ala Leu Trp Ile Ala Cys
            260                 265                 270

Val Ile Gly Tyr Leu Ala Cys Phe Ala Ser Leu Asn Ile Tyr Ile Gln
        275                 280                 285

Ser Phe Ala Arg Leu Val Trp Ser Gln Ala Gln His Asn Pro Asp His
    290                 295                 300

Tyr Leu Ala Arg Leu Ser Ser Arg His Ile Pro Asn Asn Ala Leu Asn
305                 310                 315                 320

Ala Val Leu Gly Cys Cys Val Val Ser Thr Leu Val Ile His Ala Leu
                325                 330                 335

Glu Ile Asn Leu Asp Ala Leu Ile Ile Tyr Ala Asn Gly Ile Leu Ile
            340                 345                 350

Met Ile Tyr Leu Leu Cys Met Leu Ala Gly Cys Lys Leu Leu Gln Gly
        355                 360                 365

Arg Tyr Arg Leu Leu Ala Val Val Gly Gly Leu Leu Cys Val Leu Leu
    370                 375                 380

Leu Ala Met Val Gly Trp Lys Ser Leu Tyr Ala Leu Ile Met Leu Ala
385                 390                 395                 400

Gly Leu Trp Leu Leu Leu Pro Lys Arg Lys Thr Pro Glu Asn Gly Ile
                405                 410                 415

Thr Thr



<210>    37
<211>    5803
<212>    DNA
<213>    Artificial Sequence

<220>
<223>    pDCM2


<400>    37

gttcgcttgc tgtccataaa accgcccagt ctagctatcg ccatgtaagc ccactgcaag 60

ctacctgctt tctctttgcg cttgcgtttt cccttgtcca gatagcccag tagctgacat 120

tcatccgggg tcagcaccgt ttctgcggac tggctttcta cgtgttccgc ttcctttagc 180

agcccttgcg ccctgagtgc ttgcggcagc gtgaagctag cttttatcgc cattcgccat 240

tcaggctgcg caactgttgg gaagggcgat cggtgcgggc ctcttcgcta ttacgccagc 300

tggcgaaagg gggatgtgct gcaaggcgat taagttgggt aacgccaggg ttttcccagt 360

cacgacgttg taaaacgacg gccagtgaat tcgagctcgg tacccgggga tcctctagag 420

tcgacctgca ggcatgcaag cttggcgtaa tcatggtcat agctgtttcc tgtgtgaaat 480

tgttatccgc tcacaattcc acacaacata cgagccggaa gcataaagtg taaagcctgg 540

ggtgcctaat gagtgagcta actcacatta attgcgttgc gctcactgcc cgctttccag 600

tcgggaaacc tgtcgtgcca gctgcattaa tgaatcggcc aacgcgcggg gagaggcggt 660

ttgcgtattg ggcgctcttc cgcttcctcg ctcactgact cgctgcgctc ggtcgttcgg 720

ctgcggcgag cggtatcagc tcactcaaag gcggtaatac ggttatccac agaatcaggg 780

gataacgcag gaaagaacat gtgagcaaaa ggccagcaaa aggccaggaa ccgtaaaaag 840

gccgcgttgc tggcgttttt ccataggctc cgcccccctg acgagcatca caaaaatcga 900

cgctcaagtc agaggtggcg aaacccgaca ggactataaa gataccaggc gtttccccct 960

ggaagctccc tcgtgcgctc tcctgttccg accctgccgc ttaccggata cctgtccgcc 1020

tttctccctt cgggaagcgt ggcgctttct caatgctcac gctgtaggta tctcagttcg 1080

gtgtaggtcg ttcgctccaa gctgggctgt gtgcacgaac cccccgttca gcccgaccgc 1140

tgcgccttat ccggtaacta tcgtcttgag tccaacccgg taagacacga cttatcgcca 1200

ctggcagcag ccactggtaa caggattagc agagcgaggt atgtaggcgg tgctacagag 1260

ttcttgaagt ggtggcctaa ctacggctac actagaagga cagtatttgg tatctgcgct 1320

ctgctgaagc cagttacctt cggaaaaaga gttggtagct cttgatccgg caaacaaacc 1380

accgctggta gcggtggttt ttttgtttgc aagcagcaga ttacgcgcag aaaaaaagga 1440

tctcaagaag atcctttgat cttttctacg gggtctgacg ctcagtggaa cgaaaactca 1500

cgttaaggga ttttggtcat gagattatca aaaaggatct tcacctagat ccttttgggg 1560

tgggcgaaga actccagcat gagatccccg cgctggagga tcatccagcc ctgatagaaa 1620

cagaagccac tggagcacct caaaaacacc atcatacact aaatcagtaa gttggcagca 1680

tcacccgacg cactttgcgc cgaataaata cctgtgacgg aagatcactt cgcagaataa 1740

ataaatcctg gtgtccctgt tgataccggg aagccctggg ccaacttttg gcgaaaatga 1800

gacgttgatc ggcacgtaag aggttccaac tttcaccata atgaaataag atcactaccg 1860

ggcgtatttt ttgagttatc gagattttca ggagctgata gaaacagaag ccactggagc 1920

acctcaaaaa caccatcata cactaaatca gtaagttggc agcatcaccc gacgcacttt 1980

gcgccgaata aatacctgtg acggaagatc acttcgcaga ataaataaat cctggtgtcc 2040

ctgttgatac cgggaagccc tgggccaact tttggcgaaa atgagacgtt gatcggcacg 2100

taagaggttc caactttcac cataatgaaa taagatcact accgggcgta ttttttgagt 2160

tatcgagatt ttcaggagct ctttggcatc gtctctcgcc tgtcccctca gttcagtaat 2220

ttcctgcatt tgcctgtttc cagtcggtag atattccaca aaacagcagg gaagcagcgc 2280

ttttccgctg cataaccctg cttcggggtc attatagcga ttttttcggt atatccatcc 2340

tttttcgcac gatatacagg attttgccaa agggttcgtg tagactttcc ttggtgtatc 2400

caacggcgtc agccgggcag gataggtgaa gtaggcccac ccgcgagcgg gtgttccttc 2460

ttcactgtcc cttattcgca cctggcggtg ctcaacggga atcctgctct gcgaggctgg 2520

ccggctaccg ccggcgtaac agatgagggc aagcggatgg ctgatgaaac caagccaacc 2580

aggaagggca gcccacctat caaggtgtac tgccttccag acgaacgaag agcgattgag 2640

gaaaaggcgg cggcggccgg catgagcctg tcggcctacc tgctggccgt cggccagggc 2700

tacaaaatca cgggcgtcgt ggactatgag cacgtccgcg agggcgtccc ggaaaacgat 2760

tccgaagccc aacctttcat agaaggcggc ggtggaatcg aaatctcgtg atggcaggtt 2820

gggcgtcgct tggtcggtca tttcgaaaaa ggttaggaat acggttagcc atttgcctgc 2880

ttttatatag ttcantatgg gattcacctt tatgttgata agaaataaaa gaaaatgcca 2940

ataggatatc ggcattttct tttgcgtttt tatttgttaa ctgttaattg tccttgttca 3000

aggatgctgt ctttgacaac agatgttttc ttgcctttga tgttcagcag gaagctcggc 3060

gcaaacgttg attgtttgtc tgcgtagaat cctctgtttg tcatatagct tgtaatcacg 3120

acattgtttc ctttcgcttg aggtacagcg aagtgtgagt aagtaaaggt tacatcgtta 3180

ggcggatcaa gatccatttt taacacaagg ccagttttgt tcagcggctt gtatgggcca 3240

gttaaagaat tagaaacata accaagcatg taaatatcgt tagacgtaat gccgtcaatc 3300

gtcatttttg atccgcggga gtcagtgaac aggtaccatt tgccgttcat tttaaagacg 3360

ttcgcgcgtt caatttcatc tgttactgtg ttagatgcaa tcagcggttt catcactttt 3420

ttcagtgtgt aatcatcgtt tagctcaatc ataccgagag cgccgtttgc taactcagcc 3480

gtgcgttttt tatcgctttg cagaagtttt tgactttctt gacggaagaa tgatgtgctt 3540

ttgccatagt atgctttgtt aaataaagat tcttcgcctt ggtagccatc ttcagttcca 3600

gtgtttgctt caaatactaa gtatttgtgg cctttatctt ctacgtagtg aggatctctc 3660

agcgtatggt tgtcgcctga gctgtagttg ccttcatcga tgaactgctg tacattttga 3720

tacgtttttc cgtcaccgtc aaagattgat ttataatcct ctacaccgtt gatgttcaaa 3780

gagctgtctg atgctgatac gttaacttgt gcagttgtca gtgtttgttt gccgtaatgt 3840

ttaccggaga aatcagtgta gaataaacgg atttttccgt cagatgtaaa tgtggctgaa 3900

cctgaccatt cttgtgtttg gtcttttagg atagaatcat ttgcatcgaa tttgtcgctg 3960

tctttaaaga cgcggccagc gtttttccag ctgtcaatag aagtttcgcc gactttttga 4020

tagaacatgt aaatcgatgt gtcatccgca tttttaggat ctccggctaa tgcaaagacg 4080

atgtggtagc cgtgatagtt tgcgacagtg ccgtcagcgt tttgtaatgg ccagctgtcc 4140

caaacgtcca ggccttttgc agaagagata tttttaattg tggacgaatc aaattcagaa 4200

acttgatatt tttcattttt ttgctgttca gggatttgca gcatatcatg gcgtgtaata 4260

tgggaaatgc cgtatgtttc cttatatggc ttttggttcg tttctttcgc aaacgcttga 4320

gttgcgcctc ctgccagcag tgcggtagta aaggttaata ctgttgcttg ttttgcaaac 4380

tttttgatgt tcatcgttca tgtctccttt tttatgtact gtgttagcgg tctgcttctt 4440

ccagccctcc tgtttgaaga tggcaagtta gttacgcaca ataaaaaaag acctaaaata 4500

tgtaaggggt gacgccaaag tatacacttt gccctttaca cattttaggt cttgcctgct 4560

ttatcagtaa caaacccgcg cgatttactt ttcgacctca ttctattaga ctctcgtttg 4620

gattgcaact ggtctatttt cctcttttgt ttgatagaaa atcataaaag gatttgcaga 4680

ctacgggcct aaagaactaa aaaatctatc tgtttctttt cattctctgt attttttata 4740

gtttctgttg catgggcata aagttgcctt tttaatcaca attcagaaaa tatcataata 4800

tctcatttca ctaaataata gtgaacggca ggtatatgtg atgggttaaa aaggatcacc 4860

ccagagtccc gctcagaaga actcgtcaag aaggcgatag aaggcgatgc gctgcgaatc 4920

gggagcggcg ataccgtaaa gcacgaggaa gcggtcagcc cattcgccgc caagctcttc 4980

agcaatatca cgggtagcca acgctatgtc ctgatagcgg tccgccacac ccagccggcc 5040

acagtcgatg aatccagaaa agcggccatt ttccaccatg atattcggca agcaggcatc 5100

gccatgggtc acgacgagat cctcgccgtc gggcatccgc gccttgagcc tggcgaacag 5160

ttcggctggc gcgagcccct gatgctcttc gtccagatca tcctgatcga caagaccggc 5220

ttccatccga gtacgtgctc gctcgatgcg atgtttcgct tggtggtcga atgggcaggt 5280

agccggatca agcgtatgca gccgccgcat tgcatcagcc atgatggata ctttctcggc 5340

aggagcaagg tgagatgaca ggagatcctg ccccggcact tcgcccaata gcagccagtc 5400

ccttcccgct tcagtgacaa cgtcgagaca gctgcgcaag gaacgcccgt cgtggccagc 5460

cacgatagcc gcgctgcctc gtcttggagt tcattcaggg caccggacag gtcggtcttg 5520

acaaaaagaa ccgggcgccc ctgcgctgac agccggaaca cggcggcatc agagcagccg 5580

attgtctgtt gtgcccagtc atagccgaat agcctctcca cccaagcggc cggagaacct 5640

gcgtgcaatc catcttgttc aatcatgcga aacgatcctc atcctgtctc ttgatcagat 5700

cttgatcccc tgcgccatca gatccttggc ggcaagaaag ccatccagtt tactttgcag 5760

ggcttcccaa ccttaccaga gggcgcccca gctggcaatt ccg 5803

## Description

### [Technical Field]

The present disclosure relates to a variant of YjeH, which is an inner membrane protein, and a method for producing a target product using same.

### [Background Art]

O-Acetylhomoserine acts as a precursor of methionine, which is one of the essential amino acids in the human body. Homoserine, as a precursor of both methionine and threonine, is known to be converted into threonine via phosphohomoserine and converted into methionine via acetylhomoserine.

Methionine is used not only as an additive for feeds and foods but also as a raw ingredient for infusion solutions and pharmaceutical drugs and is produced by chemical and biological synthesis. A two-step process for producing L-methionine from an L-methionine precursor, which was produced by fermentation, via enzymatic conversion was published (International Publication No. WO 2008/013432).

In the two-step process, O-succinylhomoserine and O-acetylhomoserine are used as methionine precursors, and it is very important to produce O-acetylhomoserine with high yields for the purpose of economical mass production.

### [Disclosure]

### [Technical Problem]

As a result of intensive efforts to increase production of O-acetylhomoserine or homoserine, the present inventors have found a variant of an inner membrane protein having enhanced O-acetylhomoserine or homoserine exporting activity, thereby completing the present disclosure.

### [Technical Solution]

The present disclosure provides a variant of YjeH, which is an inner membrane protein, including i) substitution of an amino acid corresponding to the 92^{nd} position with asparagine, ii) substitution of an amino acid corresponding to the 351^{st} position with leucine, or iii) substitution of both i) and ii), in an amino acid sequence of SEQ ID NO: 1, and having a homology of 95% or more and less than 100% with SEQ ID NO: 1.

The present disclosure provides a polynucleotide encoding the variant.

The present disclosure provides a vector including the polynucleotide.

The present disclosure provides a microorganism including at least any one of: a variant of an inner membrane protein YjeH including i) substitution of an amino acid corresponding to the 92^{nd} position with asparagine, ii) substitution of an amino acid corresponding to the 351^{st} position with leucine, or iii) substitution of both i) and ii), in an amino acid sequence of SEQ ID NO: 1, and having a homology of 95% or more and less than 100% with SEQ ID NO: 1; a polynucleotide encoding the variant; and a vector including the polynucleotide.

The present disclosure provides a method for producing a target product, the method including culturing a microorganism in a culture medium, wherein the microorganism includes at least any one of: a variant of an inner membrane protein YjeH including i) substitution of an amino acid corresponding to the 92^{nd} position with asparagine, ii) substitution of an amino acid corresponding to the 351^{st} position with leucine, or iii) substitution of both i) and ii), in an amino acid sequence of SEQ ID NO: 1, and having a homology of 95% or more and less than 100% with SEQ ID NO: 1; a polynucleotide encoding the variant; and a vector including the polynucleotide.

The present disclosure provides a method for producing methionine, the method including: producing O-acetylhomoserine by culturing a microorganism in a culture medium; and converting the O-acetylhomoserine into methionine by reacting the O-acetylhomoserine with a sulfide, wherein the microorganism includes at least any one of: a variant of an inner membrane protein YjeH including i) substitution of an amino acid corresponding to the 92^{nd} position with asparagine, ii) substitution of an amino acid corresponding to the 351^{st} position with leucine, or iii) substitution of both i) and ii), in an amino acid sequence of SEQ ID NO: 1, and having a homology of 95% or more and less than 100% with SEQ ID NO: 1; a polynucleotide encoding the variant; and a vector including the polynucleotide.

The present disclosure provides a method for producing glufosinate, the method including: producing O-acetylhomoserine or homoserine by culturing a microorganism in a culture medium; and converting the O-acetylhomoserine or homoserine into glufosinate, wherein the microorganism includes at least any one of: a variant of an inner membrane protein YjeH including i) substitution of an amino acid corresponding to the 92^{nd} position with asparagine, ii) substitution of an amino acid corresponding to the 351^{st} position with leucine, or iii) substitution of both i) and ii), in an amino acid sequence of SEQ ID NO: 1, and having a homology of 95% or more and less than 100% with SEQ ID NO: 1; a polynucleotide encoding the variant; and a vector including the polynucleotide.

The present disclosure provides a composition for producing homoserine including the microorganism or a culture of the microorganism.

The present disclosure provides a composition for producing O-acetylhomoserine including the microorganism or a culture of the microorganism.

The present disclosure provides a use of the variant of an inner membrane protein YjeH for producing O-acetylhomoserine or homoserine.

The present disclosure provides a use of the microorganism for producing O-acetylhomoserine or homoserine.

### [Advantageous Effects]

O-Acetylhomoserine or homoserine may be produced with higher yields by culturing a microorganism having the ability to produce O-acetylhomoserine or homoserine modified using the variant of YjeH, which is an inner membrane protein, according to the present disclosure compared to the non-modified proteins.

### [Brief Description of Drawings]

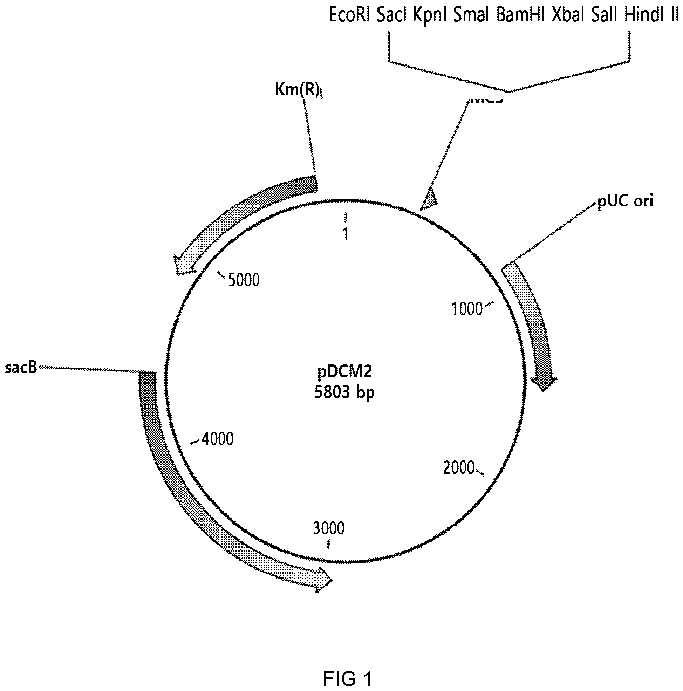

FIG. 1 is a schematic view of pDCM2 plasmid.

### [Best Mode]

The present disclosure will be described in detail. Meanwhile, each description and embodiment disclosed in the present disclosure may be applied to different descriptions and embodiments herein. In other words, all combinations of various components disclosed in the present disclosure are included within the scope of the present disclosure. Furthermore, the scope of the present disclosure should not be limited by the descriptions provided below.

An aspect of the present disclosure to achieve the above-described objects provides a variant of an inner membrane protein YjeH including i) substitution of an amino acid corresponding to the 92^{nd} position with asparagine, ii) substitution of an amino acid corresponding to the 351^{st} position with leucine, or iii) substitution of both i) and ii), in an amino acid sequence of SEQ ID NO: 1, and having a homology of 95% or more and less than 100% with SEQ ID NO: 1.

The variant of an inner membrane protein YjeH may have the ability to export O-acetylhomoserine or homoserine, i.e., O-acetylhomoserine or homoserine exporting activity.

As used herein, the term "homoserine" is an α-amino acid having a hydroxyl group in a side chain. Homoserine, as an intermediate of threonine and methionine biosynthesis in microorganisms and plants, is known to be produced from aspartic acid-4-semialdehyde and have a chemical formula of C₄H₉NO₃. Homoserine may be converted into O-acetylhomoserine by homoserine acetyl transferase in the presence of acetyl-CoA.

As used herein, the term "O-acetylhomoserine" refers to an acetyl derivative of L-homoserine, which is a specific intermediate material in the biosynthetic pathway of methionine in microorganisms. O-Acetylhomoserine is known to be produced by a reaction between homoserine and acetyl-CoA catalyzed by homoserine acetyltransferase. It has a chemical formula of C₆H₁₁NO₄. O-Acetylhomoserine may also be named O-acetyl-L-homoserine.

Specifically, in the variant of the protein according to the present disclosure, i) an amino acid corresponding to the 92^{nd} position may be substituted with a different amino acid, ii) an amino acid corresponding to the 351^{st} position may be substituted with a different amino acid, or iii) both of the amino acids corresponding to 92^{nd} position and 351^{st} position may be substituted with different amino acids, in the amino acid sequence of SEQ ID NO: 1, without being limited thereto. Substitution of the amino acids may be i) substitution of an amino acid corresponding to the 92^{nd} position with asparagine, or ii) substitution of an amino acid corresponding to the 351^{st} position with leucine, and more specifically, the variant of the protein may be a variant of the inner membrane protein YjeH in which i) the amino acid corresponding to the 92^{nd} position is substituted with asparagine, ii) the amino acid corresponding to the 351^{st} position is substituted with leucine, or iii) the amino acid corresponding to the 92^{nd} position is substituted with asparagine and the amino acid corresponding to the 351^{st} position is substituted with leucine, in the amino acid sequence of SEQ ID NO: 1, without being limited thereto.

As used herein, the term "inner membrane protein" refers to a protein as a member of the Amino Acid Efflux (AAE) Family within the Amino Acid-Polyamine-Organocation (APC) superfamily of transporters, mediating export of *O-*acetylhomoserine and/or homoserine. The protein is predicted to contain 12 transmembrane α helices, 10 of which form an inverted repeat fold that is characteristic of the APC superfamily. The inner membrane protein may be, for example, a protein including an amino acid sequence of SEQ ID NO: 1. The protein including the amino acid sequence of SEQ ID NO: 1 may be used interchangeably with protein having the amino acid sequence of SEQ ID NO: 1 or protein consisting of the amino acid sequence of SEQ ID NO: 1. In the present disclosure, the inner membrane protein may be used interchangeably with O-acetylhomoserine- or homoserine-exporting protein, protein having the ability to export O-acetylhomoserine or homoserine, protein having the O-acetylhomoserine or homoserine exporting activity, YjeH protein, or YjeH.

In the present disclosure, SEQ ID NO: 1 may refer to an amino acid sequence of YjeH, which is an inner membrane protein. Specifically, the SEQ ID NO: 1 may be an amino acid sequence of an inner membrane protein encoded by *yjeH* gene and having the O-acetylhomoserine or homoserine exporting activity. The amino acid sequence of SEQ ID NO: 1 may be obtained from a known database of the NCBI GenBank. For example, the amino acid sequence may be derived from *Escherichia coli (E. coli),* but is not limited thereto, and any sequence having the activity identical to that of the amino acid sequence may be included without limitation. In addition, although the inner membrane protein is defined as a protein including an amino acid sequence of SEQ ID NO: 1 in the present disclosure, the inner membrane protein does not exclude a mutation that may occur naturally or by addition of a meaningless sequence upstream or downstream of the amino acid sequence of the SEQ ID NO: 1, or a silent mutation thereof. As long as the protein has activity identical or equivalent to that of the protein comprising the amino acid sequence of SEQ ID NO: 1, it obviously belongs to the inner membrane protein of the present disclosure. For example, the inner membrane protein of the present disclosure may be a protein consisting of the amino acid sequence of SEQ ID NO: 1 or an amino acid sequence having at least 80%, 85%, 90%, 95%, 96%, 97%, 98%, 99%, 99.5%, or 99.7% homology or identity therewith. Also, it will be obvious that any protein having the amino acid sequence including deletion, modification, substitution, or addition in part of the amino acid sequence is within the scope of the present disclosure as long as the amino acid sequence retains the above-described homology or identity and effects equivalent to those of the protein.

That is, although the expressions "protein or polypeptide having an amino acid sequence set forth in a predetermined SEQ ID NO" and "protein or polypeptide including an amino acid sequence set forth in a predetermined SEQ ID NO" are used in the present disclosure, it is obvious that any protein having the amino acid sequence including deletion, modification, substitution, or addition in part of the amino acid sequence may also be used in the present disclosure, as long as the protein has the activity identical or equivalent to the polypeptide consisting of the predetermined amino acid sequence. For example, it is obvious that a "protein including the amino acid sequence of SEQ ID NO: 1" or "polypeptide including the amino acid sequence of SEQ ID NO: 1" belongs to a "protein consisting of the amino acid sequence of SEQ ID NO: 1" or "polypeptide consisting of the amino acid sequence of SEQ ID NO: 1" as long as the former has the same or equivalent activity to the latter.

As used herein, the term "variant" refers to a protein obtained by conservative substitution and/or modification of at least one amino acid different from that of the recited sequence while retaining functions or properties thereof. The variant may have an amino acid sequence different from the identified sequence due to substitution, deletion, or addition of several amino acids. Such variants may generally be identified by modifying at least one amino acid of the above amino acid sequence of the protein and evaluating properties of the modified protein. That is, the ability of the variant may be enhanced, may not be changed, or may be reduced relative to native protein. In addition, some variants may include variants from which at least one portion such as an N-terminal leader sequence or a transmembrane domain has been removed. Other variants may include variants in which a portion has been removed from the N- and/or C-terminus of a mature protein. The term "variant" may also be used interchangeably with other terms such as modification, modified protein, modified polypeptide, mutant, mutein, and divergent, and any terms used to indicate variation may also be used without limitation. In view of the objects of the present disclosure, the ability of the variant may be enhanced relative to a native or non-modified protein, without being limited thereto.

As used herein, the term "conservative substitution" refers to a substitution of one amino acid with another amino acid having a similar structural and/or chemical property. For example, the variant may include at least one conservative substitution while retaining at least one biological activity. Such amino acid substitution may generally occur based on similarity of polarity, charge, solubility, hydrophobicity, hydrophilicity, and/or amphipathic nature of a residue. For example, electrically charged amino acids with side chains include positively charged (basic) amino acids such as arginine, lysine, and histidine and negatively charged (acidic) amino acids such as glutamic acid and aspartic acid; and uncharged amino acids with side chains include nonpolar amino acids such as glycine, alanine, valine, leucine, isoleucine, methionine, phenylalanine, tryptophan, and proline, and polar or hydrophilic amino acids such as serine, threonine, cysteine, tyrosine, asparagine, and glutamine. Among the non-polar amino acids, aromatic amino acids include phenylalanine and tryptophan.

Variants may also include deletion or addition of amino acids that have minimal influence on properties and a secondary structure of a polypeptide. For example, a polypeptide may be conjugated to a signal (or leader) sequence at the N-terminus of a protein which co-translationally or post-translationally directs transfer of the protein. The polypeptide may also be conjugated with another sequence or linker to identify, purify, or synthesize the polypeptide.

In the present disclosure, the "substituted with a different amino acid" is not particularly limited as long as the amino acid after substitution is different from the amino acid before substitution.

In the present disclosure, the "substituted with a different amino acid" is not particularly limited as long as the amino acid after substitution is different from the amino acid before substitution. Specifically, the amino acid may be substituted with any one of lysine, histidine, glutamic acid, aspartic acid, glycine, alanine, valine, leucine, isoleucine, methionine, phenylalanine, tryptophan, proline, serine, threonine, cysteine, tyrosine, asparagine, and glutamine different from the previous amino acid sequence.

Specifically, in the amino acid sequence of SEQ ID NO: 1, phenylalanine, which is an amino acid corresponding to the 92^{nd} position, may be substituted with asparagine, or phenylalanine, which is an amino acid corresponding to the 351^{st} position, may be substituted with leucine. Meanwhile, in the present disclosure, it is obvious that the expression "substituted with a certain amino acid" means that the amino acid after substitution is different from the amino acid before substitution unless the expression "substituted with a different amino acid" is used.

Also, the substituted amino acid residue may include not only a natural amino acid, but also a non-natural amino acid. The non-natural amino acid may be, for example, a D-amino acid, (homo)-amino acid, (*beta*-homo)-amino acid, *N*-methyl amino acid, α-methyl amino acid, and uncommon amino acid (e.g., citrulline or naphthylalanine), but is not limited thereto. Meanwhile, in the present disclosure, it is obvious that the expression "substituted with a certain amino acid" means that the amino acid after substitution is different from the amino acid before substitution unless the expression "substituted with a different amino acid" is used.

The variant may be one in which at least any one of the amino acids corresponding to the 92^{nd} position and the 351^{st} position is substituted with an amino acid different from that before substitution in the amino acid sequence of SEQ ID NO: 1, or substituted with an uncharged amino acid with a side chain different from that before substitution, but is not limited thereto.

Specifically, the variant may be a variant of the protein in which i) the amino acid corresponding to the 92^{nd} position is substituted with a different amino acid, ii) the amino acid corresponding to the 351^{st} position is substituted with a different amino acid, or iii) the amino acids corresponding to the 92^{nd} position and the 351^{st} position are substituted with different amino acids, in the amino acid sequence of SEQ ID NO: 1, without being limited thereto. The substitution of amino acids may be i) substitution of the 92^{nd} amino acid with asparagine, or ii) substitution of the 351^{st} amino acid with leucine. More specifically, the variant may be a variant of the inner membrane protein YjeH in which i) the 92^{nd} amino acid is substituted with asparagine, ii) the 351^{st} amino acid is substituted with leucine, or iii) the 92^{nd} amino acid is substituted with asparagine and the 351^{st} amino acid is substituted with leucine, in the amino acid sequence of SEQ ID NO: 1, without being limited thereto.

As used herein, the term "corresponding to" refers to an amino acid residue at a position cited in a protein or polypeptide or an amino acid residue similar, identical, or homologous to the residue cited in the protein or polypeptide. As used herein, the term "corresponding region" generally refers to a region similar thereto in a related protein or a reference protein.

In the present disclosure, a particular numbering may be used for a position of an amino acid residue of the protein used in the present disclosure. For example, a position corresponding to an amino acid residue of the protein of the present disclosure may be renumbered by aligning an amino acid sequence of a protein to be compared and the amino acid sequence of the protein of the present disclosure.

The variant of an inner membrane protein YjeH provided in the present disclosure may refer to a variant having enhanced ability to export O-acetylhomoserine or homoserine compared to non-modified protein by substituting an amino acid at a specific position in the above-described inner membrane protein having the O-acetylhomoserine or homoserine exporting ability.

The variant of an inner membrane protein YjeH including i) substitution of the amino acid corresponding to the 92^{nd} position with asparagine, ii) substitution of the amino acid corresponding to the 351^{st} position with leucine, or substitution of both i) and ii), in amino acid sequence of SEQ ID NO: 1 may include any one of the amino acid sequences of SEQ ID NOS: 35 and 36, specifically consisting essentially of any one of the amino acid sequences of SEQ ID NOS: 35 and 36, more specifically consisting of any one of the amino acid sequences of SEQ ID NOS: 35 and 36, without being limited thereto.

In addition, the variant may include an amion acid sequence of any one of SEQ ID NS: 35 and 36, or an amino acid sequence in which at least any one amino acid of the 92^{nd} and 351^{st} amino acids is fixed in the amino acid sequences of SEQ ID NOS: 35 and 36 and having at least 80% homology with the amino acid sequences of SEQ ID NOS: 35 and 36, without being limited thereto. Specifically, variant of the present disclosure may include a polypeptide having at least 80%, 90%, 95%, 96%, 97%, 98%, or 99% homology or identity with any one of the amino acid sequences of SEQ ID NOS: 35 and 36. Also, it is obvious that any protein having the amino acid sequence including deletion, modification, substitution, or addition in part of the amino acid sequence at a position other than the 92^{nd} position and the 351^{st} position is within the a scope of the present disclosure as long as the protein retains the above-described homology or identity and effects equivalent to those of the variant.

] As used herein, the term "homology" or "identity" refers to a degree of relevance between two given amino acid sequences or base sequences and may be expressed as a percentage. These terms homology and identity may often be used interchangeably.

Sequence homology or identity of conserved polynucleotides or polypeptides may be determined by standard alignment algorithm, and default gap penalties established by a program may be used together therewith. Substantially, homologous or identical sequences may hybridize with each other at least about 50%, 60%, 70%, 80%, or 90% of the entire sequence or the entire length under moderate or highly stringent conditions. In hybridized polynucleotides, polynucleotides including a degenerate codon instead of a codon may also be considered.

The sequence homology, similarity, or identity between two given polypeptides or polynucleotides may be determined using any known computer algorism such as "FASTA" program by using default parameters as introduced by, for example, Pearson et al. (1988) Proc. Natl. Acad. Sci. USA 85:2444. Alternatively, Needleman-Wunsch algorithm (1970, J. Mol. Biol. 48:443-453) performed in a Needleman program of The European Molecular Biology Open Software Suite (EMBOSS) package (Rice et al., 2000, Trends Genet. 16:276-277) (version 5.0.0 or later) may be used to determine the same (including GCG program package (Devereux, J. et al., Nucleic Acids Research 12:387 (1984)), BLASTP, BLASTN, FASTA (Atschul, S. F. et al., J MOLEC BIOL 215:403 (1990); Guide to Huge Computers, Martin J. Bishop, ed., Academic Press, San Diego,1994, and CARILLO et al. (1988) SIAM J Applied Math 48:1073). For example, the homology, similarity, or identity may be determined using BLAST, from The National Center for Biotechnology Information database, or ClustalW.

The homology, similarity, or identity between polynucleotides or polypeptides may be determined, for example, by comparing sequence information using a GAP computer program, such as a program introduced by Needleman et al. (1970), J Mol Biol. 48:443, for example, as disclosed in Smith and Waterman, Adv. Appl. Math (1981) 2:482. In brief, the GAP program defines similarity as the number of aligned symbols (i.e., nucleotides or amino acids) which are similar, divided by the total number of symbols in a shorter of two sequences. Default parameters for the GAP program may include: (1) a binary comparison matrix (containing a value of 1 for identity and 0 for non-identity) a weighted comparison matrix of Gribskov et al. (1986) Nucl. Acids Res. 14:6745 disclosed in Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) a penalty of 3.0 for each gap and an additional 0.10 penalty for each symbol in each gap (or gap open penalty of 10, and a gap extension penalty of 0.5); and (3) no penalty for end gaps.

Also, the sequence homology, similarity, or identity between two given polynucleotides or polypeptides may be identified by comparing sequences thereof by southern hybridization under defined stringent conditions, and the defined stringent hybridization conditions are within the scope of the technology and may be defined by a method well known to one of ordinary skill in the art (For example, J. Sambrook et al., Molecular Cloning, A Laboratory Manual, 2nd Edition, Cold Spring Harbor Laboratory press, Cold Spring Harbor, New York, 1989; F. M. Ausubel et al., Current Protocols in Molecular Biology, John Wiley & Sons, Inc., New York).

As used herein, the term "variant of an inner membrane protein" or "variant of an inner membrane protein YjeH " may have the ability to export O-acetylhomoserine or homoserine and may be used interchangeably with a modified polypeptide having the ability to produce O-acetylhomoserine or homoserine, O-acetylhomoserine- or homoserine-producing modified polypeptide, modified polypeptide having the O-acetylhomoserine or homoserine producing ability, modified polypeptide having the O-acetylhomoserine or homoserine exporting activity, variant with the *O-*acetylhomoserine or homoserine exporting activity, O-acetylhomoserine- or homoserine-exporting variant, modified homoserine-exporting protein, modified *O-*acetylhomoserine-exporting protein, modified YjeH, variant YjeH, YjeH variant, YjeH mutant, or the like.

In addition, the protein may be derived from stains belonging to the genus *Escherichia,* the genus *Erwinia,* the genus *Serratia,* the genus *Providencia,* the genus *Corynebacterium,* the genus *Pseudomonas,* the genus *Leptospira,* the genus *Salmonella,* the genus *Brevibacterium,* the genus *Hypomononas,* the genus *Chromobacterium,* and the genus *Norcardia,* or fungi or yeast, specifically derived from strains belonging to the genus *Escherichia,* the genus *Corynebacterium,* and the genus *Leptospira,* and yeast, and more specifically derived from strains belonging to the genus *Escherichia, e.g., Escherichia coli* (*E*. *coli),* without being limited thereto.

The variant of an inner membrane protein YjeH may include mutation at the 92^{nd} position and/or the 351^{st} position in the amino acid sequence of SEQ ID NO: 1 and any variant including addition or deletion of an amino acid to or from the amino acid sequence of SEQ ID NO: 1 may be within the scope of the present disclosure as long as the amino acid corresponding to the 92^{nd} position and/or the 351^{st} position from the N-terminus of SEQ ID NO: 1 is substituted. The variant of an inner membrane protein YjeH may include an amino acid sequence including substitution of the 92^{nd} amino acid and/or 351^{st} amino acid with a different amino acid in the amino acid sequence of SEQ ID NO: 1 and may have enhanced activity compared to the inner membrane protein including the amino acid sequence of SEQ ID NO: 1 or not including mutation. The variant of an inner membrane protein YjeH may have a homology or identity of 80%, 85%, 90%, 95%, 96%, 97%, 98%, 99%, 99.5%, 99.7%, or more and less than 100% with SEQ ID NO: 1.

Mutation of the 92^{nd} or 351^{st} amino acid may be substitution of the 92^{nd} amino acid with asparagine or substitution of the 351^{st} amino acid with leucine.

Specifically, the variant of an inner membrane protein YjeH may include i) substitution of the 92^{nd} amino acid with asparagine, ii) substitution of the 351^{st} amino acid with leucine, or substitution of both i) and ii), in the amino acid sequence of SEQ ID NO: 1 and may have enhanced activity compared to the protein including the amino acid sequence of SEQ ID NO: 1 or the inner membrane protein derived from a wild-type microorganism before modification.

Another aspect of the present disclosure provides a polynucleotide encoding the variant of an inner membrane protein YjeH.

The inner membrane protein YjeH and the variant thereof are as described above.

As used herein, the term "polynucleotide" refers to a DNA or RNA strand having a certain minimum length or more as a polymer of nucleotides in which nucleotide monomers are linked to each other in the form of a long chain by covalent bonds, more specifically the polynucleotide may be a polynucleotide fragment encoding the variant.

The polynucleotide may include any nucleotide sequence encoding the variant of an inner membrane protein YjeH of the present disclosure without limitation. In the present disclosure, a gene encoding the amino acid sequence of the inner membrane protein YjeH is *yjeH* gene, and the gene may be derived from *Escherichia coli (E. coli),* without being limited thereto.

Specifically, the polynucleotide of the present disclosure may include various modifications made in a coding region provided not to change an amino acid sequence of a polypeptide expressed from the coding region by codon degeneracy or in consideration of codons preferred by a living organism in which the protein is expressed. Specifically, any polynucleotide sequence encoding the variant of an inner membrane protein YjeH in which the 92^{nd} or 351^{st} amino acid is substituted with a different amino acid in the amino acid sequence of SEQ ID NO: 1 may be included without limitation.

In addition, any sequence encoding an inner membrane protein, in which the 92^{nd} or 351^{st} amino acid of the amino acid sequence of SEQ ID NO: 1 is substituted with a different amino acid by hybridizing with a probe that may be prepared from known gene sequences, for example, sequences complementary to all or part of the nucleotide sequence under stringent conditions, may be included without limitation. The term "stringent conditions" refers to conditions which permit specific hybridization between polynucleotides. Such conditions are disclosed in detail in known documents (e.g., J. Sambrook *et al.*)*.* For example, the conditions may include conditions for performing hybridization between genes having a high homology or identity, e.g., a homology or identity of 40% or more, specifically 90% or more, more specifically 95% or more, even more specifically 97% or more, and most specifically 99% or more, without performing hybridization between genes having a homology lower than the above homologies or identities, or performing hybridization once, specifically two or three times, under conventional washing conditions for Southern hybridization at a salt concentration and temperature of 60°C, 1×SSC, and 0.1% SDS, specifically 60°C, 0.1×SSC, 0.1% SDS, and more specifically 68°C, 0.1×SSC, and 0.1% SDS.

Hybridization requires that two polynucleotides have complementary sequences, although bases may mismatch according to the degree of stringency of hybridization. The term "complementary" is used to describe the relationship between nucleotide bases capable of hybridizing with each other. For example, with respect to DNA, adenine is complementary to thymine, and cytosine is complementary to guanine. Thus, the present disclosure may include not only a substantially similar nucleic acid sequence but also a nucleic acid fragment isolated but complementary to the entire sequence.

Specifically, the polynucleotides having homology or identity may be detected using the above-described hybridization conditions and using a hybridization process at a Tₘ value of 55°C. Also, the Tₘ value may be, but is not limited to, 60°C, 63°C, or 65°C, and may be appropriately adjusted by those skilled in the art according to the intended purposes.

An appropriate degree of stringency for hybridization of polynucleotides may depend on lengths of the polynucleotides and a degree of complementarity and parameters thereof are well known in the art (Sambrook *et al., supra,* 9.50-9.51, 11.7-11.8).

Another aspect of the present disclosure provides a vector including a polynucleotide encoding the variant of an inner membrane protein YjeH.

The inner membrane protein YjeH, the variant thereof, and the polynucleotide are as described above.

The vector of the present disclosure may be a DNA construct containing a nucleotide sequence of a polynucleotide encoding a target polypeptide and operably linked to a suitable expression regulatory region (expression regulatory sequence) so as to be able to express the target polypeptide in a suitable host cell. The expression regulatory sequence may include a promoter capable of initiating transcription, any operator sequence for regulating the transcription, a sequence encoding a suitable mRNA ribosome binding site, and a sequence for regulating termination of transcription and translation. When a suitable host cell is transformed with the vector, the vector may replicate or function independently from the host genome, or may integrate into genome thereof.

As used herein, the term "expression" includes any process related to producing a polypeptide such as transcription, post-transcriptional modification, translation, post-translational modification, and secretion, *etc.* without being limited thereto.

As used herein, the term "expression vector" refers to a linear or circular nucleic acid molecule including a coding sequence and an operably linked regulatory sequence for expression thereof.

As used herein, the term "operably linked" refers to a placing of a regulatory sequence to an appropriate position to regulate expression a coding sequence. Thus, "operably linked" includes a binding between a regulatory region of a functional domain having a known or desired activity such as a promoter, a stop codon, a signal sequence, or an enhancer and a target (gene or polypeptide) to regulate expression, secretion, or function of the target in accordance with the known or desired activity.

The vector used in the present disclosure is not particularly limited, and any known vector may be used. Examples of the known vectors may include natural or recombinant plasmids, cosmids, viruses, and bacteriophages. For example, pWE15, M13, MBL3, MBL4, IXII, ASHII, APII, t10, t11, Charon4A, and Charon21A, *etc.* may be used as a phage vector or cosmid vector, and pBR-based, pUC-based, pBluescriptll-based, pGEM-based, pTZ-based, pCL-based, and pET-based vectors may be used as a plasmid vector. Specifically, pDCM2, pACYC177, pACYC184, pCL, pECCG117, pUC19, pBR322, pMW118, and pCC1BAC vectors may be used. More specifically, the vector used in the present disclosure may be pDCM2 (FIG. 1, SEQ ID NO: 37) constructed for insertion and substitution of a gene in the chromosome of *Corynebacterium,* but is not limited thereto, and any known expression vector may be used.

For example, a polynucleotide encoding a target polypeptide may be inserted into a chromosome using a vector for chromosomal insertion into cells. The insertion of the polynucleotide into the chromosome may be performed by any method known in the art, for example, homologous recombination, without being limited thereto. The polynucleotide may further include a selection marker to confirm the chromosomal insertion. The selection marker is used to select cells that are transformed with the vector, that is, to confirm insertion of a desired nucleic acid molecule, and examples of the selection marker may include markers providing selectable phenotypes, such as drug tolerance, nutrient requirement, resistance to cytotoxic agents, or expression of surface polypeptide. Only cells expressing the selection marker are able to survive or to show different phenotypes under the environment treated with a selective agent, and thus the transformed cells may be selected.

Another aspect of the present disclosure provides a microorganism including at least any one of: a variant of an inner membrane protein YjeH including i) substitution of an amino acid corresponding to the 92^{nd} position with asparagine, ii) substitution of an amino acid corresponding to the 351^{st} position with leucine, or iii) substitution of both i) and ii), in an amino acid sequence of SEQ ID NO: 1, and having a homology of 95% or more and less than 100% with SEQ ID NO: 1; a polynucleotide encoding the variant; and a vector including the polynucleotide.

The SEQ ID NO: 1, the inner membrane protein YjeH, the variant thereof, the polynucleotide encoding the variant, and the vector including the polynucleotide are as described above.

As used herein, the term, "microorganism including the variant of an inner membrane protein YjeH" refers to a microorganism with enhanced ability to export O-acetylhomoserine or homoserine prepared by introducing the variant of an inner membrane protein YjeH into a microorganism having weak ability to export O-acetylhomoserine or homoserine. Specifically, the microorganism is a microorganism expressing the variant of an inner membrane protein YjeH including at least one amino acid modification in the amino acid sequence of SEQ ID NO: 1, and the amino acid modification may include substitution of the 92^{nd} or 351^{st} amino acid from the N-terminus with a different amino acid.

In view of the objects of the present disclosure, the extracellular export of *O-*acetylhomoserine or homoserine of the microorganism including at least any one of the variant of an inner membrane protein YjeH; the polynucleotide encoding the variant; and the vector including the polynucleotide increases compared to wild-type or non-modified microorganisms, and thus the microorganism is characterized in that the ability to producing O-acetylhomoserine or homoserine is enhanced. While wild-type or non-modified microorganisms cannot export or can produce trace amounts of O-acetylhomoserine or homoserine, the ability of producing O-acetylhomoserine or homoserine may be enhanced according to the present disclosure by increasing the export amounts of O-acetylhomoserine or homoserine by introducing at least any one of the variant of an inner membrane protein YjeH; the polynucleotide encoding the variant; and the vector including the polynucleotide.

As used herein, the term "non-modified microorganism" does not exclude strains including mutation that may occur naturally in microorganisms and refers to a wild-type strain or a microorganism not including the variant of an inner membrane protein YjeH. The "non-modified microorganism" may be used interchangeably with "strain before modification", "microorganism before modification", "non-mutated strain", "non-mutated microorganism", "non-modified strain", or "reference microorganism".

As used herein, the term a protein "to be expressed/expressed" means a state in which a target protein is introduced into or modified to be expressed in a microorganism. In the case where the target protein is present in the microorganism, the activity of the protein may be enhanced compared to the activity of endogenous protein thereof or that before modification. In view of the objects of the present disclosure, the "target protein" may be the above-described variant of an inner membrane protein YjeH.

Specifically, the term "introduction of a protein" refers to providing activity of a particular protein to a microorganism which does not possess the protein or enhancing the activity of the protein compared to the intrinsic activity of the protein or the activity before modification. For example, the introduction of a protein may refer to introduction of a polynucleotide encoding the particular protein into chromosome or introduction of a vector including the polynucleotide encoding the particular protein into a microorganism, thereby expressing the activity of the protein. In addition, the "enhancement of activity" may mean that the activity of a particular protein of a microorganism is enhanced when compared with the intrinsic activity or the activity before modification. The term "intrinsic activity" may refer to activity of a particular protein possessed by a parent strain before transformation when a microorganism is transformed by natural or artificial genetic variation.

Specifically, the enhancement of the activity of the present disclosure may be achieved by way of at least one method selected from the group consisting of: a method of increasing the number of copies of a gene encoding the variant protein; a method of introducing mutation into an expression regulatory sequence of a gene encoding the variant protein; a method of replacing the expression regulatory sequence of the gene with a sequence encoding the variant protein and having stronger activity; a method of replacing a gene encoding a wild-type inner membrane protein on the chromosome with a gene encoding the variant protein; a method of additionally introducing mutation into a gene encoding the variant protein to enhance the activity of the variant protein; and a method of introducing the variant protein into the microorganism, without being limited thereto.

In the above description, the increasing the number of copies of the gene, although not particularly limited thereto, may be performed in a state operably linked to a vector, or by being inserted into the chromosome within a host cell. Specifically, the increase in gene copy number may be performed by introducing a vector, to which a polynucleotide encoding the variant protein of the present disclosure is operably linked, and which may replicate and function irrespective of a host cell, into a cell of the host. Alternatively, a vector, to which the polynucleotide is operably linked, capable of inserting the polynucleotide into the chromosome of the host cell, is introduced into the host cell. The insertion of the polynucleotide into the chromosome may be performed using a known method in the art, for example, by homologous recombination.

The modification of the expression regulatory sequence to increase expression level of the polynucleotide may be performed by, but is not particularly limited to, inducing mutation on the sequence by deletion, insertion, non-conservative substitution, conservative substitution, or any combination thereof to further enhance the activity of the expression regulatory sequence or replacement with a sequence having stronger activity. The expression regulatory sequence may include, but is not limited to, a promoter, an operator sequence, a sequence encoding a ribosome binding site, and a sequence regulating termination of transcription and translation.

A stronger promoter than the intrinsic promoter may be linked upstream of the polynucleotide expression unit, without being limited thereto. Examples of the stronger promoter known in the art may include CJ1 to CJ7 promoters (US 7662943 B2), lac promoter, trp promoter, trc promoter, tac promoter, Lambda phage PR promoter, PL promoter, tet promoter, gapA promoter, SPL7 promoter, SPL13 (sm3) promoter (US 10584338 B2), O2 promoter (US 10273491 B2), tkt promoter, and yccA promoter, without being limited thereto.

In addition, the modification of the polynucleotide sequence on the chromosome may be performed by inducing mutation in the expression regulatory sequence by deletion, insertion, non-conservative substitution, conservative substitution, or any combination thereof to further enhance the activity of the polynucleotide sequence, or by replacing the nucleotide sequence with a nucleotide sequence modified to have a stronger activity, without being limited thereto.

The introduction of the protein and enhancement of the activity may increase the activity or concentration of the protein from the activity or concentration of a wild-type or non-modified microorganism strain, without being limited thereto.

In the present disclosure, the microorganism including the variant of an inner membrane protein YjeH may be a recombinant microorganism prepared by transformation using a vector including a polynucleotide encoding the variant of an inner membrane protein YjeH, but is not limited thereto.

As used herein, the term "transformation" refers to a process of introducing a vector including a polynucleotide encoding a target protein into a host cell in such a way that the protein encoded by the polynucleotide is expressed in the host cell. The transformed polynucleotide may be either in a form inserted into the chromosome of the host cell or in a form located outside the chromosome as long as the protein is expressed in the host cell. In addition, the polynucleotide includes DNA and/or RNA encoding the target protein. The polynucleotide may be introduced into the host cell in any form as long as the polynucleotide is introduced into the host cell and the protein is expressed therein. For example, the polynucleotide may be introduced into the host cell in the form of an expression cassette that is a gene construct including all of the essential elements required for self-expression. The expression cassette may generally include a promoter, a transcription termination signal, a ribosome binding site, and a translation termination signal which are operably linked to the polynucleotide. The expression cassette may be in the form of a self-replicable expression vector. Also, the polynucleotide may be introduced into the host cell in its original form and operably linked to a sequence required for the expression in the host cell, without being limited thereto.

Types of the microorganism having the homoserine producing ability are not particularly limited as long the microorganism is able to produce O-acetylhomoserine or homoserine, and specifically the microorganism may be any microorganism belonging the genus *Corynebacterium,* the genus *Escherichia,* the genus *Enterobacter,* the genus *Erwinia,* the genus *Serratia,* the genus *Providencia,* and the genus *Brevibacterium,* more specifically a microorganism belonging to the genus *Corynebacterium.*

More specifically, the microorganism belonging to the genus *Corynebacterium* may be *Corynebacterium glutamicum, Corynebacterium ammoniagenes, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium pollutisoli, Cxorynebacterium imitans, Corynebacterium testudinoris,* or *Corynebacterium flavescens,* and any microorganisms belonging to the genus *Corynebacterium* may be used without limitation.

In the present disclosure, a parent strain of the microorganism may be a microorganism in which a gene weakening the biosynthesis pathway of O-acetylhomoserine or homoserine is additionally inactivated or the biosynthesis pathway of O-acetylhomoserine or homoserine is enhanced to increase production of O-acetylhomoserine or homoserine, without being limited thereto.

Specifically, to inactivate the gene weakening the biosynthesis pathway of O-acetylhomoserine or homoserine, for example, expression of *metB* gene (SEQ ID NO: 17) encoding cystathionine gamma-synthase involved in a degradation pathway of O-acetylhomoserine or *metY* gene (SEQ ID NO: 22) encoding *O-*acetylhomoserine (thiol)-lyase involved in a degradation pathway of *O-*acetylhomoserine may be weakened or inactivated.

To enhancing the biosynthesis pathway of O-acetylhomoserine or homoserine, for example, genetic mutation may be introduced into *lysC* gene (SEQ ID NO: 27) encoding aspartokinase, or expression of *metX* gene (SEQ ID NO: 32) encoding O-acetylhomoserine transferase may be amplified.

However, the embodiment is not limited thereto, the ability to produce O-acetylhomoserine or homoserine may be enhanced by any known methods for gene expression regulation.

As used herein, the term "enhancement/increase" is a concept including an increase in the activity compared to the intrinsic activity.

The enhancement or increase of gene activity may be achieved by application of various methods well known in the art. Examples of the methods may include at least any one selected from the group consisting of: a method of increasing the copy number of a gene; a method of introducing mutation into an expression regulatory sequence of the gene; a method of replacing the expression regulatory sequence of the gene with a sequence having stronger activity; a method of additionally introducing mutation into the gene in order to enhance the activity of the gene; a method of introducing a foreign gene into a microorganism; and any combination thereof, without being limited thereto.

As used herein, the term "inactivation" of a protein means that the activity of the protein is weakened compared to the intrinsic activity, or that the protein has no activity.

The inactivation of the protein may be achieved by way of various methods well known in the art. Examples of the methods may include: a method of deleting a part or the entirety of a gene encoding the protein on the chromosome including removing the activity of the protein; a method of replacing the gene encoding the protein on the chromosome with a mutated gene to reduce the enzymatic activity; a method of introducing mutation into an expression regulatory sequence of a gene encoding the protein on the chromosome; a method of replacing the expression regulatory sequence of the gene encoding the protein with a sequence having a weak activity or no activity (e.g., a method of replacing a promoter of the gene with a promoter weaker than the endogenous promoter); a method of deleting a part or the entirety of the gene encoding the protein on the chromosome; a method of introducing an antisense oligonucleotide (e.g., antisense RNA), which inhibits translation from an mRNA into the protein via a complementary binding to a transcript of the gene on the chromosome; a method of making the attachment of a ribosome impossible by forming a secondary structure by artificially adding a complementary sequence to the Shine-Dalgarno (SD) sequence on the upstream of the SD sequence of the gene encoding the protein; and a reverse transcription engineering (RTE) method, which adds a promoter for reverse transcription to the 3' terminal of the open reading frame (ORF) of the corresponding sequence, and a combination thereof, but are not particularly limited thereto.

Another aspect of the present disclosure provides a method for producing a target product including culturing a microorganism in a culture medium, wherein the microorganism includes at least any one of: a variant of an inner membrane protein YjeH including i) substitution of an amino acid corresponding to the 92^{nd} position with asparagine, ii) substitution of an amino acid corresponding to the 351^{st} position with leucine, or iii) substitution of both i) and ii), in an amino acid sequence of SEQ ID NO: 1, and having a homology of 95% or more and less than 100% with SEQ ID NO: 1; a polynucleotide encoding the variant; and a vector including the polynucleotide.

The SEQ ID NO: 1, the inner membrane protein YjeH, the variant thereof, the polynucleotide encoding the variant, the vector including the polynucleotide, and the microorganism including the same are as described above.

The target product may be O-acetylhomoserine or homoserine, but is not limited thereto.

As used herein, the term "culturing" refers to growing the microorganism in an appropriately adjusted environment. A culture process of the present disclosure may be performed according to an appropriate medium and culturing conditions known in the art. The culture process may be easily adjusted for use by a skilled person in the art according to a strain to be selected. The culturing of the microorganism may be performed in a batch process, a continuous process, a fed-batch process, *etc.,* without being limited thereto.

As used herein, the term "culture medium" refers to a substance in which nutrients required for culturing the microorganism are mixed as main ingredients and supplies nutrients and growth factors as well as water which are essential for survival and growth. Specifically, although culture media and other culturing conditions for culturing the microorganism of the present disclosure are not particularly limited as long as the culture media are commonly used in culturing microorganisms, the microorganism of the present disclosure may be cultured in an ordinary medium containing appropriate carbon sources, nitrogen sources, phosphorus sources, inorganic compounds, amino acids, and/or vitamins under aerobic conditions while adjusting temperature, pH, and the like.

In the present disclosure, as the carbon sources, carbohydrates such as glucose, fructose, sucrose, and maltose; sugar alcohols such as mannitol and sorbitol; organic acids such as pyruvic acid, lactic acid, and citric acid; and amino acids such as glutamic acid, methionine, and lysine may be used. In addition, natural organic nutrients such as starch hydrolysates, molasses, blackstrap molasses, rice bran, cassava, sugar cane bagasse, and corn steep liquor may be used, and specifically carbohydrates such as glucose and sterile pretreated molasses (i.e., molasses converted to reduced sugars) may be used, and suitable amounts of any other carbon sources may also be used without limitation. These carbon sources may be used alone or in a combination of at least two thereof.

As the nitrogen sources, inorganic nitrogen sources such as ammonia, ammonium sulfate, ammonium chloride, ammonium acetate, ammonium phosphate, ammonium carbonate, and ammonium nitrate; and organic nitrogen sources such as amino acids, e.g., glutamic acid, methionine, and glutamine, peptone, NZ-amine, meat extract, yeast extract, malt extract, corn steep liquor, casein hydrolysate, fish or degradation products thereof, and defatted soybean cake or degradation products thereof may be used. These nitrogen sources may be used alone or in a combination of at least two thereof, without being limited thereto.

As the phosphorus sources, monopotassium phosphate, dipotassium phosphate, or sodium-containing salts corresponding thereto may be used. As inorganic compounds, sodium chloride, calcium chloride, iron chloride, magnesium sulfate, iron sulfate, manganese sulfate, calcium carbonate, and the like may be used. Also, amino acids, vitamins, and/or appropriate precursor may further be included. The culture medium and precursors may be added to the culture medium in a batch or continuous process, without being limited thereto.

In the present disclosure, during the culturing process of the microorganism, compounds such as ammonium hydroxide, potassium hydroxide, ammonia, phosphoric acid, and sulfuric acid may be properly added to the cultures in order to adjust the pH of the culture medium. Also, a defoaming agent such as fatty acid polyglycol ester may be added during culturing in order to inhibit formation of foams. In addition, oxygen or oxygen-containing gas may be injected into the culture medium to maintain the culture medium in an aerobic condition, and nitrogen, hydrogen, or carbon dioxide gases may be injected into the cultures to maintain the culture in anaerobic and micro-aerobic conditions without injecting any other gases therefor, but the embodiment is not limited thereto.

The temperature of the culture medium may be maintained at 25°C to 50°C, more specifically at 30°C to 37°C, without being limited thereto. The culturing may be continued until a desired amount of a product is obtained, specifically for 10 hours to 100 hours, without being limited thereto.

The target product produced by the culturing may be exported into the culture medium or remain in the cells.

The method may further include recovering the target product from the culture medium or the microorganism.

The recovering of the target product produced in the culturing step of the present disclosure may be performed by collecting the target product from the culture using any known method selected according to the culturing method. For example, centrifugation, filtration, anion-exchange chromatography, crystallization, and high-performance liquid chromatography (HPLC) may be used, and the target amino acid may be recovered from the culture medium or the microorganism using any appropriate method in the art, without being limited thereto. The culture may also be named culture broth.

Also, the recovering step may include a purification process which may be performed using an appropriate method well known in the art. Thus, the recovered target product may be a purified form or a fermentation broth of a microorganism including the target product *(*Introduction to Biotechnology and Genetic Engineering, A. J. Nair., 2008).

Another aspect of the present disclosure provides a method for producing methionine, the method including: producing O-acetylhomoserine by culturing a microorganism in a culture medium; and converting the O-acetylhomoserine into methionine by reacting the O-acetylhomoserine with a sulfide, wherein the microorganism includes at least any one of: a variant of an inner membrane protein YjeH including i) substitution of an amino acid corresponding to the 92^{nd} position with asparagine, ii) substitution of an amino acid corresponding to the 351^{st} position with leucine, or iii) substitution of both i) and ii), in an amino acid sequence of SEQ ID NO: 1, and having a homology of 95% or more and less than 100% with SEQ ID NO: 1; a polynucleotide encoding the variant; and a vector including the polynucleotide.

The SEQ ID NO: 1, the inner membrane protein YjeH, the variant thereof, the polynucleotide encoding the variant, the vector including the polynucleotide, the microorganism including the same, and O-acetylhomoserine are as described above.

In the present disclosure, O-acetylhomoserine, as a methionine precursor, may be converted into methionine via reaction with a sulfide.

The reaction with the sulfide refers to a process of generating L-methionine from O-succinyl homoserine using any known method. For example, L-methionine may be produced by reacting methyl mercaptan, as a sulfide, with O-acetylhomoserine. In addition, a catalyst or an enzyme may be added or reaction may be performed in a microorganism including an enzyme to increase reaction rates and increase yields.

The "sulfide" may be, for example, methyl mercaptan, and the methyl mercaptan may be any methyl mercaptan derivatives capable of providing sulfur atoms such as dimethylsulfide (DMS) disclosed in International Publication No. WO 2010/098629) as well as sodium methyl mercaptan (CH₃S-Na) in a liquid phase and methyl mercaptan (CH₃SH) in a gaseous or liquid state.

As an enzyme used in methionine conversion reaction, cystathionine-γ-synthase, O-acetylhomoserine sulfhydrylase, or O-succinylhomoserine sulfhydrylase which are known in the art may be used.

A process of methionine conversion reaction of the present disclosure may be expressed as the following reaction scheme:

O-acetyl-L-homoserine + CH₃SH ↔ L-methionine + acetate

In the reaction, a CH₃S- residue of methyl mercaptan is substituted with an acetate residue of O-acetylhomoserine to produce L-methionine.

The reaction may be conducted at a temperature of 20°C to 45°C while maintaining the pH of a reaction medium in the range of 6 to 7, and pyridoxal 5'-phosphate may be added as a coenzyme.

The conversion of an L-methionine precursor into L-methionine in the presence of methyl mercaptan is an enzymatic reaction and is disclosed in International Publication No. WO 2008/013432. International Publication No. WO 2008/013432 provides details on properties and preparation of methionine-converting enzymes having an appropriate activity for converting an L-methionine precursor into L-methionine in the presence of methyl mercaptan. Such appropriate methionine-converting enzyme may be prepared using gene expression according to biotechnological methods.

A sequence of a gene encoding an enzyme having methionine converting activity may be obtained from, but not limited to, database of the NCBI (USA) and DNA data bank (KEGG, Japan). For biological conversion, the gene may be cloned from the obtained gene sequence, and then introduced into an expression vector. Specifically, the gene may be expressed in an active form from pCL-CJI vector (CJ, Korea) that is an expression vector for *E. coli* in recombinant strains, and an expressed protein may be obtained from an enzyme solution in which sonicated cells are lysed. Both the strain expressing the methionine-converting enzyme and the expressed methionine-converting enzyme may be directly used in the methionine conversion reaction.

The methionine-converting enzyme expressed from the gene or the microorganism strain expressing the same may be directly, partially, or entirely mixed with a fermentation supernatant or a fermentation broth in which an L-methionine precursor is accumulated to initiate the reaction. That is, the reaction may be initiated by adding the methionine-converting enzyme or the microorganism strain expressing the same to the fermentation solution in which O-acetylhomoserine is accumulated and adding methyl mercaptan thereto.

Specifically, O-acetylhomoserine that is accumulated in the fermentation solution may be converted into L-methionine by cystathionine-γ-synthase, *O-*acetylhomoserine sulfhydrylase, or O-succinyl homoserine sulfhydrylase.

The methionine conversion reaction may be confirmed using DTNB (5,5-dithiobis(2-nitro-benzoic acid), Sigma, USA), and the reaction product may be analyzed by HPLC. During the process of methionine conversion reaction, byproducts such as acetic acid may be additionally obtained, without a separate production process, via reaction between methyl mercaptan and O-acetylhomoserine.

The enzymatic reaction between L-methionine precursor and methyl mercaptan may be represented by the following reaction scheme:

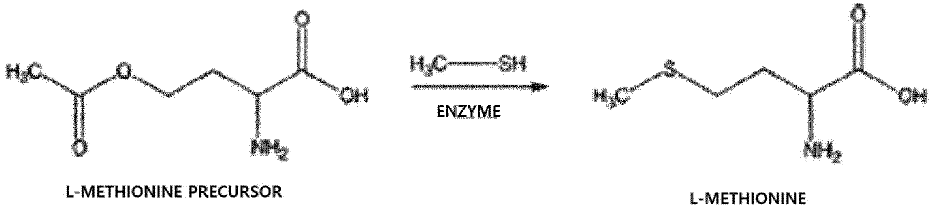

The method of producing methionine may be easily determined by one or ordinary skill in the art under known optimal culturing conditions and enzyme activation conditions. Specific culturing methods and media are as described above.

Another aspect of the present disclosure provides a method for producing glufosinate, the method including: producing O-acetylhomoserine or homoserine by culturing a microorganism in a culture medium; and converting the O-acetylhomoserine or homoserine into glufosinate, wherein the microorganism includes at least any one of: a variant of an inner membrane protein YjeH including i) substitution of an amino acid corresponding to the 92^{nd} position with asparagine, ii) substitution of an amino acid corresponding to the 351^{st} position with leucine, or iii) substitution of both i) and ii), in an amino acid sequence of SEQ ID NO: 1, and having a homology of 95% or more and less than 100% with SEQ ID NO: 1; a polynucleotide encoding the variant; and a vector including the polynucleotide.

The SEQ ID NO: 1, the inner membrane protein YjeH, the variant thereof, the polynucleotide encoding the variant, the vector including the polynucleotide, the microorganism including the same, and homoserine are as described above.

As used herein, the term "glufosinate" refers to a broad range of naturally occurring herbicides produced by several species of *Streptomyces* soil bacteria, and is also known as phosphinothricin.

In the present disclosure, O-acetylhomoserine or homoserine may be converted into glufosinate via a plurality of steps including halogenation.

The glufosinate conversion reaction is disclosed, for example, in CN 108516991 A. Specifically, the glufosinate conversion reaction may include: (1) a step of performing azeotropic dehydration of homoserine (Formula II) using an organic solvent in the presence of an acidic catalyst; and a step of adding a halogenating agent to the azeotropically dehydrated homoserine and performing halogenation to obtain L-3,6-bis(2-haloethyl)-2,5-dione piperazine (Formula III); (2) a step of adding methylphosphinic acid diester or methyl phosphite diester to the L-3,6-bis(2-haloethyl)-2,5-dione piperazine and performing an Abbov reaction using a high-boiling solvent in the presence of a catalyst to obtain a compound of Formula IV; and (3) a step of performing a hydrolysis reaction by dissolving the compound of Formula IV in an acid and heating the solution, removing a solvent after completion of the reaction, adding an alcohol thereto, and performing conversion reaction by adding an alkylene oxide thereto to obtain glufosinate (Formula I).

The glufosinate conversion reaction using homoserine as a precursor may be represented by the following reaction scheme:

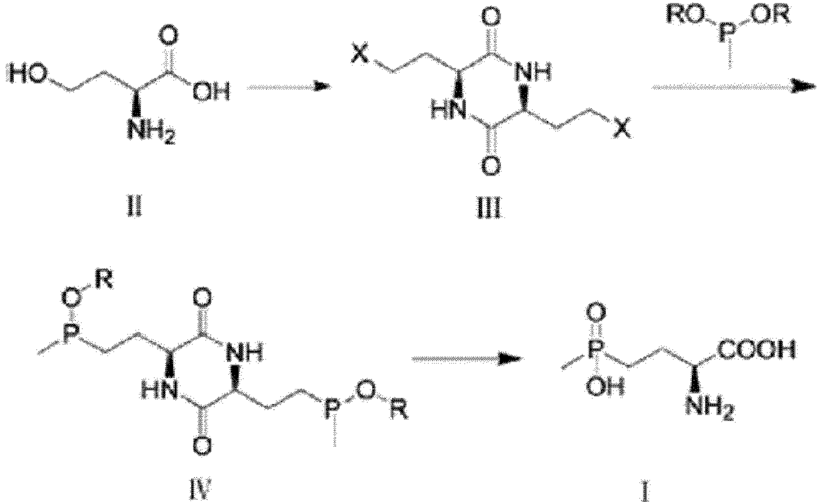

The method for producing glufosinate may be performed using homoserine prepared by biological fermentation as a precursor according to the present disclosure. Glufosinate may be produced by preparing L-3,6-bis(2-haloethyl)-2,5-dione piperazine by reaction with methylphosphonium by azeotropic dehydration and halogenation, performing an Abbov reaction, and then performing a hydrolysis reaction as described above.

Another aspect of the present disclosure provides a composition for producing homoserine including a microorganism or a culture of the microorganism, wherein the microorganism includes at least any one of: a variant of an inner membrane protein YjeH including i) substitution of an amino acid corresponding to the 92^{nd} position with asparagine, ii) substitution of an amino acid corresponding to the 351^{st} position with leucine, or iii) substitution of both i) and ii), in an amino acid sequence of SEQ ID NO: 1, and having a homology of 95% or more and less than 100% with SEQ ID NO: 1; a polynucleotide encoding the variant; and a vector including the polynucleotide.

Another aspect of the present disclosure provides a composition for producing O-acetylhomoserine including a microorganism or culture of the microorganism, wherein the microorganism includes at least any one of: a variant of an inner membrane protein YjeH including i) substitution of an amino acid corresponding to the 92^{nd} position with asparagine, ii) substitution of an amino acid corresponding to the 351^{st} position with leucine, or iii) substitution of both i) and ii), in an amino acid sequence of SEQ ID NO: 1, and having a homology of 95% or more and less than 100% with SEQ ID NO: 1; a polynucleotide encoding the variant; and a vector including the polynucleotide.

The SEQ ID NO: 1, the inner membrane protein YjeH, the variant thereof, the polynucleotide encoding the variant, the vector including the polynucleotide, the microorganism including the same, homoserine, and O-acetylhomoserine are as described above.

The composition for producing O-acetylhomoserine or homoserine may refer to a composition used to produce O-acetylhomoserine or homoserine by the variant of an inner membrane protein YjeH of the present disclosure. The composition may include the variant of an inner membrane protein YjeH or a component capable of operating the variant of an inner membrane protein YjeH without limitation. The variant of an inner membrane protein YjeH may be in a form inserted into a vector capable of expressing an operably linked gene in a host cell.

The composition may further include a cryoprotectant or an excipient. The cryoprotectant or the excipient may be a naturally occurring substance or one which does not occur naturally, but is not limited thereto. As another example, the cryoprotectant or the excipient may be a substance that the microorganism does not naturally come into contact with or a substance that is not naturally included in the microorganism, but is not limited thereto.

Another aspect of the present disclosure provides a use of the variant of an inner membrane protein YjeH for producing O-acetylhomoserine or homoserine.

Another aspect of the present disclosure provides a use of a microorganism for producing O-acetylhomoserine or homoserine, wherein the microorganism includes at least any one of: a variant of an inner membrane protein YjeH including i) substitution of an amino acid corresponding to the 92^{nd} position with asparagine, ii) substitution of an amino acid corresponding to the 351^{st} position with leucine, or iii) substitution of both i) and ii), in an amino acid sequence of SEQ ID NO: 1, and having a homology of 95% or more and less than 100% with SEQ ID NO: 1; a polynucleotide encoding the variant; and a vector including the polynucleotide.

The homoserine, O-acetylhomoserine, inner membrane protein YjeH, the variant thereof, the polynucleotide encoding the variant, the vector including the polynucleotide and the microorganism including the same are as described above.

### [Mode for Invention]

Hereinafter, the present disclosure will be described in more detail with reference to the following examples. However, the following examples are merely presented to exemplify the present disclosure, and the scope of the present disclosure is not limited thereto.

### Example 1: Construction of Plasmid

A plasmid (pDCM2, FIG. 1, SEQ ID NO: 37) was designed for insertion and replacement of a gene in the *Corynebacterium* chromosome and synthesized using a gene synthesis service of Bionics Co., Ltd. The plasmid was designed to contain a restriction enzyme efficient for cloning with reference to a paper related to the *sacB* system commonly known in the art (Gene, 145 (1994) 69-73). The synthesized pDCM2 plasmid has the following characteristics.
1) Due to a replication origin only active in *E*. *coli,* the plasmid self-replicates only in *E*. *coli* and cannot self-replicate in *Corynebacterium.*
2) The plasmid contains a kanamycin-resistance gene as a selection marker.
3) The plasmid contains Levan sucrose gene (sacB) as a secondary positive-selection marker.
4) No gene information derived from the pDCM2 plasmid remains in the ultimately prepared strain.

### Example 2: Construction of Strains Introduced with Variant Exogenous Membrane Protein (YjeH) and Evaluation of Ability to Produce O-Acetylhomoserine and Homoserine

### 2-1. Construction of Strains Introduced with Exogenous Membrane

### Protein (YjeH) and Variant YjeH

In order to evaluate effectiveness of YjeH that is introduced into *Corynebacterium glutamicum* ATCC13032 as an exogenous membrane protein and an O-acetylhomoserine-exporting protein, a vector for chromosomal insertion including *yjeH* gene (SEQ ID NO: 2) encoding YjeH derived from *E. coli* was prepared.

Specifically, in order to construct a transposase-deletion vector, a pair of primers (SEQ ID NOS: 4 and 5) for amplifying a 5' upstream region and a pair of primers (SEQ ID NOS: 6 and 7) for amplifying a 3' downstream region with respect to a gene encoding transposase (SEQ ID NO: 3, gene No. NCgl2335) were designed. The primers of SEQ ID NOS: 5 and 6 were designed to cross each other. A restriction enzyme Smal sequence was located in this region. The primer sequences are shown in Table 1 below.

**Table 1**

| SEQ ID NO: | Sequence name | Sequence |
|---|---|---|
| SEQ ID NO: 4 | Tn_5 F | tgaattcgagctcggtacccCACCGACGCGCATCTG CCT |
| SEQ ID NO: 5 | Tn_5 R | GGTGTGGTGACTTTCAGCAGTTCCCGGGG GGGAGGAGGCATGTGGTGTTG |
| SEQ ID NO: 6 | Tn_3 F | CAACACCACATGCCTCCTCCCCCCCGGGA ACTGCTGAAAGTCACCACACC |
| SEQ ID NO: 7 | Tn_3 R | gtcgactctagaggatccccCTCCCAAACCATTGAG GAATGG |

PCR was performed using the chromosome of wild-type (WT) ATCC13032 as a template and primers of SEQ ID NOS: 4 and 5 and SEQ ID NOS: 6 and 7. The PCR was performed under the following conditions. After denaturation at 95°C for 5 minutes, 30 cycles of denaturation at 95°C for 30 seconds, annealing at 55°C for 30 seconds, and polymerization at 72°C for 30 seconds were repeated, and then polymerization was performed at 72°C for 7 minutes. As a result DNA fragments of an 851 bp 5' upstream region and an 847 bp 3' downstream region with respect to the NCgl2335 gene deletion region were obtained.

PCR was performed using the two amplified DNA fragments as templates and primers of SEQ ID NOS: 4 and 7. PCR was performed under the following conditions. After denaturation at 95°C for 5 minutes, 30 cycles of denaturation at 95°C for 30 seconds, annealing at 55°C for 30 seconds, and polymerization at 72°C for 90 seconds were repeated, and then polymerization was performed at 72°C for 7 minutes. As a result, a 1648 bp DNA fragment including a region capable of deleting the gene encoding transposase (SEQ ID NO: 3, gene No. NCgl2335) was amplified.

The obtained PCR products were fusion-cloned using a pDCM2 vector treated with a restriction enzyme Smal and an In-Fusion^{®} HD Cloning Kit (Clontech). *E. coli* DH5α was transformed with the cloned vector, and the transformed *E. coli* was smeared on an LB solid medium containing kanamycin (25 mg/L). Colonies transformed with the plasmid inserted with the target gene were selected by PCR, and the plasmid was obtained using a plasmid extraction method. Finally, pDCM2-ΔNCgl2335 recombinant vector including the cloned NCgl2335 deletion cassette was constructed.

In order to evaluate effectiveness of an O-acetylhomoserine-exporting protein, a vector for chromosomal insertion including a gene (SEQ ID NO: 2) encoding YjeH derived from *E. coli* was prepared. To this end, a vector expressing *yjeH* gene was constructed using the CJ7 promoter (SEQ ID NO: 8, US 7662943 B2). A pair of primers (SEQ ID NOS: 9 and 10) for amplifying the CJ7 promoter region and a pair of primers (SEQ ID NOS: 11 and 12) for amplifying the *yjeH* region of *E. coli* were designed. The primer sequences are shown in Table 2 below.

**Table 2**

| SEQ ID NO: | Sequence name | Sequence |
|---|---|---|
| SEQ ID NO: 9 | CJ7_yjeH F | ACACCACATGCCTCCTCcccAGAAACATCCC AGCGCTAC |
| SEQ ID NO: 10 | CJ7_yjeH R | AGTTCTTGTTTGAGTCCACTCATAGTGTTTC CTTTCGTTGGGT |
| SEQ ID NO: 11 | yjeH F | ACCCAACGAAAGGAAACACTATGAGTGGA CTCAAACAAGAACTG |
| SEQ ID NO: 12 | yjeH R | GACTTTCAGCAGTTcccgggTTATGTGGTTAT GCCATTTTCCGG |

PCR was performed using pECCG117-PCJ7-gfp (US 7662943 B2) as a template and primers of SEQ ID NOS: 9 and 10 and PCR was performed using the chromosome of wild-type *E. coli* as a template and primers of SEQ ID NOS: 11 and 12, respectively. The PCR was performed under the following conditions. After denaturation at 95°C for 5 minutes, 30 cycles of denaturation at 95°C for 30 seconds, annealing at 55°C for 30 seconds, and polymerization at 72°C for 90 seconds were repeated, and then polymerization was performed at 72°C for 7 minutes. As a result, a 360 bp DNA fragment of the CJ7 promoter region and a 1297 bp DNA fragment of the *yjeH* gene of *E. coli* were obtained.

PCR was performed using the two amplified DNA fragments as templates and primers of SEQ ID NOS: 9 and 12. The PCR was performed under the following conditions. After denaturation at 95°C for 5 minutes, 30 cycles of denaturation at 95°C for 30 seconds, annealing at 55°C for 30 seconds, and polymerization at 72°C for 90 seconds were repeated, and then polymerization was performed at 72°C for 7 minutes. As a result, a 1614 bp DNA fragment including a region into which the CJ7 promoter and the *yjeH* gene were inserted was amplified.

The gene-deletion DNA fragment obtained by the PCR was cloned to pDCM2-ANCgl2335 vector treated with a restriction enzyme Smal using an In-Fusion^{®} HD Cloning Kit (Clontech). Finally, pDCM2-ΔNCgl2335::PCJ7-yjeH (eco, WT) recombinant vector was constructed.

Specifically, phenylalanine (Phe), which is the 92^{nd} amino acid of the amino acid sequence of YjeH, was substituted with asparagine (Asn) (F92N) using the pDCM2-ΔNCgl2335::PCJ7-yjeH (eco, WT) plasmid as a template and primers of SEQ ID NOS: 13 and 14. A constructed plasmid including a gene encoding the variant YjeH (F92N) was named pDCM2-ΔNCgl2335::PCJ7-yjeH (eco, F92N).

Also, phenylalanine (Phe), which is the 351^{st} amino acid of the amino acid sequence of YjeH, was substituted with leucine (F351L) using the pDCM2-ΔNCgl2335::PCJ7-yjeH (eco, WT) plasmid as a template and primers of SEQ ID NOS: 15 and 16. A constructed plasmid including a gene encoding the variant YjeH (F351L) was named pDCM2-ΔNCgl2335::PCJ7-yjeH (eco, F351L). The primer sequences are shown in Table 3 below.

**Table 3**

| SEQ ID NO: | Sequence name | Sequence |
|---|---|---|
| SEQ ID NO: 13 | F92N F | CGGCTGGCTGAATTTATCGGTCA |
| SEQ ID NO: 14 | F92N R | TGACCGATAAATTCAGCCAGCCG |
| SEQ ID NO: 15 | F351L F | CAATGGCATCCTTATTATGATTT |
| SEQ ID NO: 16 | F351L R | AAATCATAATAAGGATGCCATTG |

The ATCC13032 strain was transformed with each of the constructed pDCM2-ΔNCgl2335, pDCM2-ΔNCgl2335::PCJ7-yjeH (eco, WT), pDCM2-ΔNCgl2335::PCJ7-yjeH (eco, F92N), and pDCM2-ΔNCgl2335::PCJ7-yjeH (eco, F351L) using an electric-pulse method and subjected to a secondary crossover process to obtain ATCC13032 ΔNCgl2335, ATCC13032 ΔNCgl2335::PCJ7-yjeH (eco, WT), ATCC13032 ΔNCgl2335::PCJ7-yjeH (eco, F92N), and ATCC13032 ΔNCgl2335::PCJ7-yjeH (eco, F351L) in which the NCgl2335 gene was deleted from the chromosome. Insertion of inactivated NCgl2335 gene and newly introduced *E. coli yjeH* gene was identified by performing PCR using the primers of SEQ ID NOS: 4 and 7 and comparing results with those of ATCC13032 in which the NCgl2335 gene was not inactivated.

### 2-2. Evaluation of Ability to Produce O-Acetylhomoserine

In order to compare the O-acetylhomoserine (O-AH) producing ability of ATCC13032 ΔNCgl2335, ATCC13032 ΔNCgl2335::PCJ7-yjeH (eco, WT), ATCC13032 ΔNCgl2335::PCJ7-yjeH (eco, F92N), and ATCC13032 ΔNCgl2335::PCJ7-yjeH (eco, F351L), which were constructed in Example 2-1, with that of the wild-type strain ATCC13032, O-acetylhomoserine contained in the cultures was analyzed according to the following method.

One inoculation loop of each of the strains was inoculated to a 250 mL corner-baffled flask containing 25 mL of a medium below and then cultured while shaking at 33°C for 20 hours at 200 rpm. The concentrations of O-acetylhomoserine were analyzed using HPLC, and the analyzed concentrations are shown in Table 4.

### O-Acetylhomoserine Production Medium (pH 7.2)

30 g of glucose, 2 g of KH₂PO₄, 3 g of urea, 40 g of (NH₄)2SO₄, 2.5 g of peptone, 5 g (10 mL) of corn steep liquor (CSL, Sigma), 0.5 g of MgSO₄·7H₂O, and 20 g of CaCO₃ (based on 1 L of distilled water)

**Table 4**

| Strain name | O-Acetylhomoserine (g/L) |
|---|---|
| ATCC13032 | 0.3 |
| ATCC13032 ΔNCgl2335 | 0.3 |
| ATCC13032 ΔNCgl2335::PCJ7-yjeH (eco, WT) | 0.7 |
| ATCC13032 ΔNCgl2335::PCJ7-yjeH (eco, F92N) | 1.2 |
| ATCC13032 ΔNCgl2335::PCJ7-yjeH (eco, F351L) | 1.0 |

As a result, it was confirmed that O-acetyl-L-homoserine was accumulated with a concentration of 0.3 g/L when the ATCC13032, as a control, was cultured, and production of O-acetyl-L-homoserine was not affected by deletion of the NCgl2335 gene (transposase), as shown in Table 4. Particularly, it was confirmed that O-acetyl-L-homoserine was accumulated with a concentration of 0.7 g/L when the wild-type *yjeH* gene was expressed, and O-acetyl-L-homoserine was accumulated with concentrations of 1.2 g/L and 1.0 g/L when the variant *yjeH* genes were expressed.

Therefore, based on the results shown above, it was confirmed that the wild-type and variant *yjeH* genes of the present disclosure significantly increased production of the amino acid of interest by exporting O-acetylhomoserine from the ATCC13032.

The ATCC13032 ΔNCgl2335::PCJ7-yjeH (eco, F92N) and ATCC13032 ΔNCgl2335::PCJ7-yjeH (eco, F351L) strains were named *Corynebacterium glutamicum* CM04-0651 and *Corynebacterium glutamicum* CM04-0652, respectively, and deposited at the Korean Culture Center of Microorganisms under the Budapest Treaty on December 4, 2019, with Accession Nos. KCCM12633P and KCCM12634P, respectively.

### 2-3. Evaluation of Ability to Produce Homoserine

In order to compare the homoserine producing ability of ATCC13032 ΔNCgl2335, ATCC13032 ΔNCgl2335::PCJ7-yjeH (eco, WT), ATCC13032 ΔNCgl2335::PCJ7-yjeH (eco, F92N), and ATCC13032 ΔNCgl2335::PCJ7-yjeH (eco, F351L), which were constructed in Example 2-1, with that of the wild-type strain ATCC13032, homoserine contained in the cultures was analyzed according to the following method.

One inoculation loop of each of the strains was inoculated to a 250 mL corner-baffled flask containing 25 mL of a medium below and then cultured while shaking at 33°C for 20 hours at 200 rpm. The concentrations of homoserine were analyzed using HPLC, and the analyzed concentrations are shown in Table 5.

### Homoserine Production Medium (pH 7.2)

30 g of glucose, 2 g of KH₂PO₄, 3 g of urea, 40 g of (NH₄)₂SO₄, 2.5 g of peptone, 5 g (10 mL) of corn steep liquor (CSL, Sigma), 0.5 g of MgSO₄·7H₂O, and 20 g of CaCO₃ (based on 1 L of distilled water)

**Table 5**

| Strain name | Homoserine (g/L) |
|---|---|
| ATCC13032 | 0.0 |
| ATCC13032 ΔNCgl2335 | 0.0 |
| ATCC13032 ΔNCgl2335::PCJ7-yjeH (eco, WT) | 0.0 |
| ATCC13032 ΔNCgl2335::PCJ7-yjeH (eco, F92N) | 0.2 |
| ATCC13032 ΔNCgl2335::PCJ7-yjeH (eco, F351L) | 0.2 |

As a result, it was confirmed that homoserine was not accumulated with a concentration of 0.0 g/L when the ATCC13032, as a control, was cultured, and production of homoserine was not affected by deletion of the NCgl2335 gene (transposase) as shown in Table 5. Particularly, it was also confirmed that homoserine was not accumulated with a concentration of 0.0 g/L when the wild-type *yjeH* gene was expressed, and homoserine was accumulated with concentrations of 0.2 g/L and 0.2 g/L when the variant *yjeH* genes were expressed.

Therefore, based on the results shown above, it was confirmed that the variant *yjeH* gene of the present disclosure increased production of the amino acid of interest by exporting homoserine from the ATCC13032.

### Example 3: Construction of Strain with Enhanced Ability to Produce O-Acetylhomoserine and Evaluation of Ability to Produce O-Acetylhomoserine and Homoserine

### 3-1. Inactivation of Cystathionine Gamma (γ)-synthase (MetB)

Via PCR using chromosomal DNA of *Corynebacterium glutamicum* ATCC13032 as a template, *metB* gene encoding cystathionine γ-synthase involved in a degradation pathway of O-acetylhomoserine was obtained. Information on a nucleotide sequence of the *metB* gene (NCBI No. Ncgl2360, SEQ ID NO: 17) was obtained from GenBank of the National Institutes of Health (USA), and primers (SEQ ID NOS: 18 and 19) including the N-terminus of the *metB* gene and a linker and primers (SEQ ID NOS: 20 and 21) including the C-terminus and the linker were synthesized based thereon. The primer sequences are shown in Table 6 below.

**Table 6**

| SEQ ID NO | Sequence name | Sequence |
|---|---|---|
| SEQ ID NO: 18 | metB_N_del F | TCTAGACGCCCGCATACTGGCTTC |
| SEQ ID NO: 19 | metB_N_del R | CCCATCCACTAAACTTAAACAGATGTGAT CGCCCGGC |
| SEQ ID NO: 20 | metB_C_del F | TGTTTAAGTTTAGTGGATGGGGAAGAACC ACCCAGGCC |
| SEQ ID NO: 21 | metB_C_del R | GTCGACCAATCGTCCAGAGGGCG |

PCR was performed using the chromosomal DNA of ATCC13032 as a template and primers of SEQ ID NOS: 18 and 19 and SEQ ID NOS: 20 and 21. PfuUltra^{™} high-reliability DNA polymerase (Stratagene) was used as a polymerase, and PCR was performed by repeating 30 cycles of denaturation at 96°C for 30 seconds, annealing at 53°C for 30 seconds, and polymerization at 72°C for 1 minute. As a result, an amplified 558 bp gene containing the N-terminus of the *metB* gene and the linker and an amplified 527 bp gene containing the C-terminus of the *metB* gene and the linker were obtained, respectively.

PCR was performed using the two amplified genes obtained as described above as templates by repeating 10 cycles of denaturation at 96°C for 60 seconds, annealing at 50°C for 60 seconds, and polymerization at 72°C for 1 minute, and then adding the primers of SEQ ID NOS: 18 and 21 thereto, followed by polymerization 20 times. As a result, a 1064 bp inactivation cassette including the N-terminus-linker-C-terminus of the *metB* gene was obtained. The obtained PCR products were treated with restriction enzymes Xbal and Sall and ligated to the pDCM2 vector treated with the restriction enzymes Xbal and Sall for cloning to ultimately construct a pDCM2-ΔmetB recombinant vector cloned with the metB inactivation cassette.

The ATCC13032 was transformed with the constructed pDCM2-ΔmetB vector using an electric-pulse method and subjected to a secondary crossover process to obtain ATCC13032 ΔmetB in which the *metB* gene was inactivated in the chromosome. The inactivated *metB* gene was ultimately confirmed by comparison with ATCC13032 in which the *metB* gene was not inactivated after performing PCR using the primers of SEQ ID NOS: 18 and 21.

### 3-2. Inactivation of O-Acetylhomoserine (thiol)-lyase (MetY)

Via PCR using chromosomal DNA of *Corynebacterium glutamicum* ATCC13032 as a template, *metY* gene encoding O-acetylhomoserine (thiol)-lyase involved in a degradation pathway of O-acetylhomoserine was obtained. Information on a nucleotide sequence of the *metY* gene (NCBI No. Ncgl0625, SEQ ID NO: 22) was obtained from GenBank of the National Institutes of Health (USA), and primers (SEQ ID NOS: 23 and 24) including the N-terminus of the *metY* gene and a linker and primers (SEQ ID NOS: 25 and 26) including the C-terminus and a linker were synthesized based thereon. The primer sequences are shown in Table 7 below.

**Table 7**

| SEQ ID NO | Sequence name | Sequence |
|---|---|---|
| SEQ ID NO: 23 | metY_N_del F | TCTAGACCATCCTGCACCATTTAG |
| SEQ ID NO: 24 | metY_N_del R | CCCATCCACTAAACTTAAACACGCTCCT GCCAGGTTC |
| SEQ ID NO: 25 | metY_C_del F | TGTTTAAGTTTAGTGGATGGGCTTGGTA CGCAACCAAGG |
| SEQ ID NO: 26 | metY_C_del R | GTCGACGATTGCTCCGGCTTCGG |

PCR was performed using the chromosomal DNA of ATCC13032 as a template and primers of SEQ ID NOS: 23 and 24 and SEQ ID NOS: 25 and 26. PfuUltra^{™} high-reliability DNA polymerase (Stratagene) was used as a polymerase, and PCR was performed by repeating 30 cycles of denaturation at 96°C for 30 seconds, annealing at 53°C for 30 seconds, and polymerization at 72°C for 1 minute. As a result, an amplified 548 bp gene containing the N-terminus of the *metY* gene and the linker and an amplified 550 bp gene containing the C-terminus of the *metY* gene and the linker were obtained, respectively. PCR was performed using the two amplified genes obtained as described above as templates by repeating 10 cycles of denaturation at 96°C for 60 seconds, annealing at 50°C for 60 seconds, and polymerization at 72°C for 1 minute, and then adding the primers of SEQ ID NOS: 23 and 26 thereto, followed by polymerization 20 times. As a result, a 1077 bp inactivation cassette including the N-terminus-linker-C-terminus of the *metY* gene was obtained. The obtained PCR products were treated with restriction enzymes Xbal and Sall and ligated to the pDCM2 vector treated with the restriction enzymes Xbal and Sall for cloning to ultimately construct a pDCM2-ΔmetY recombinant vector cloned with the metY inactivation cassette.

The ATCC13032 ΔmetB strain was transformed with the constructed pDCM2-ΔmetY vector using an electric-pulse method and subjected to a secondary crossover process to obtain ATCC13032 ΔmetB ΔmetY in which the *metY* gene is inactivated in the chromosome. The inactivated *metY* gene was ultimately confirmed by comparison with ATCC13032 in which the *metY* gene was not inactivated after performing PCR using the primers of SEQ ID NOS: 23 and 26.

### 3-3. Introduction of Variant Aspartokinase (LysC)

In order to maximize production of O-acetylhomoserine, *Corynebacterium glutamicum* ATCC13032-derived *lysC* gene (SEQ ID NO: 27) encoding aspartokinase was introduced with mutation (L377K) for enhancement of expression of the *lysC* gene and relief of feedback inhibition by L-lysine and L-threonine (US 10662450 B2). In order to prepare a vector including the variant *lysC* gene, a pair of primers (SEQ ID NOS: 28 and 29) for amplifying a 5' upstream region and a pair of primers (SEQ ID NOS: 30 and 31) for amplifying a 3' downstream region with respect to the mutation position were designed. Xbal and Sall restriction enzyme regions were inserted to both ends of the primers of SEQ ID NOS: 28 and 31, and the primers of SEQ ID NOS: 29 and 30 were designed to cross each other, and nucleotide substitution was located at this region. Sequences of the primers are as shown in Table 8 below.

**Table 8**

| SEQ ID NO | Sequence name | Sequence |
|---|---|---|
| SEQ ID NO: 28 | lysC_L377K_5 F | TCCTCTAGAGCTGCGCAGTGTTGAATACG |
| SEQ ID NO: 29 | lysC_L377K_5 R | AGGTGGAAATCTTTTCGATGTTC |
| SEQ ID NO: 30 | lysC_L377K_3 F | GAACATCGAAAAGATTTCCACCT |
| SEQ ID NO: 31 | lysC_L377K_3 R | GACTCTAGAGTTCACCTCAGAGACGATTA |

PCR was performed using the chromosome of *Corynebacterium glutamicum* ATCC13032 as a template and primers of SEQ ID NOS: 28 and 29 and SEQ ID NOS: 30 and 31. PCR was performed under the following conditions. After denaturation at 95°C for 5 minutes, 30 cycles of denaturation at 95°C for 30 seconds, annealing at 55°C for 30 seconds, and polymerization at 72°C for 30 seconds were repeated, and then polymerization was performed at 72°C for 7 minutes. As a result, a 512 bp DNA fragment of a 5' upstream region and a 522 bp DNA fragment of a 3' downstream region with respect to the mutation of the *lysC* gene were obtained.

PCR was performed using the two amplified DNA fragments as templates and primers of SEQ ID NOS: 28 and 31. PCR was performed under the following conditions. After denaturation at 95°C for 5 minutes, 30 cycles of denaturation at 95°C for 30 seconds, annealing at 55°C for 30 seconds, and polymerization at 72°C for 60 seconds were repeated, and then polymerization was performed at 72°C for 7 minutes. As a result, a 1011 bp DNA fragment including the variant *lysC* (L377K) gene encoding an aspartokinase variant in which leucine at the 377^{th} position was substituted with lysine was amplified.

The *lysC* (L377K) fragment obtained by the PCR was treated with restriction enzymes Xbal and Sall at both ends and ligated to the pDCM2 vector treated with the restriction enzymes Xbal and Sall, followed by cloning to ultimately obtain a pDCM2-lysC (L377K) recombinant vector cloned with *lysC* (L377K) substitution cassette.

The ATCC13032 ΔmetB ΔmetY strain was transformed with the prepared pDCM2-lysC (L377K) vector by an electric-pulse method and subjected to a second crossover process to obtain *Corynebacterium glutamicum* ATCC 13032 ΔmetB ΔmetY lysC (L377K) introduced with the variant *lysC* gene on the chromosome. The variant gene was ultimately identified by PCR performed using the primers of SEQ ID NOS: 28 and 31 and then comparing the sequence with that of the wild-type *lysC* gene by sequencing.

### 3-4. Construction of Plasmid Introduced with O-Acetylhomoserine Transferase (MetX)

In order to amplify a gene encoding O-acetylhomoserine transferase (MetX), information on a nucleotide sequence of *metX* gene (NCBI No. NCgl0624, SEQ ID NO: 32) was obtained from GenBank of the National Institutes of Health (USA), a BamHI restriction enzyme site was inserted into both ends of each of primers (SEQ ID NOS: 33 and 34) for amplification from a promoter region (located about 300 bp upstream from a start codon) to a terminator region (located about 100 bp downstream from a stop codon) based thereon. PCR was performed under the following conditions. After denaturation at 95°C for 5 minutes, 30 cycles of denaturation at 95°C for 30 seconds, annealing at 55°C for 30 seconds, and polymerization at 72°C for 90 seconds were repeated, and then polymerization was performed at 72°C for 7 minutes. As a result, a 1546 bp DNA fragment of a coding region of the *metX* gene was obtained. A pECCG117 vector (KR 10-0057684 B1) and the *metX* DNA fragment were treated with the restriction enzyme BamHI, ligated using a DNA ligase, and cloned to obtain a plasmid which was named pECCG117-metX WT. Sequences of the primers are as shown in Table 9 below.

**Table 9**

| SEQ ID NO | Sequence name | Sequence |
|---|---|---|
| SEQ ID NO: 33 | metX F | GGATCCCCTCGTTGTTCACCCAGCAACC |
| SEQ ID NO: 34 | metX R | GGATCCCAAAGTCACAACTACTTATGTTAG |

### 3-5. Construction of O-Acetylhomoserine-producing Strain and Evaluation of Ability to Produce O-Acetylhomoserine

After the pECCG117-metX WT vector prepared in Example 3-4 was introduced into *Corynebacterium glutamicum* ATCC13032 ΔmetB ΔmetY lysC (L377K) using an electric-pulse method, the strain was smeared on a selective medium containing kanamycin (25 mg/L) and cultured to obtain transformants.

For comparison of the O-acetylhomoserine producing ability of the strains, the strains were cultured by way of the following method, and O-acetylhomoserine contained in the cultures was analyzed.

One inoculation loop of each of the strains was inoculated to a 250 mL corner-baffled flask containing 25 mL of a medium below and then cultured while shaking at 37°C for 20 hours at 200 rpm. The concentrations of O-acetylhomoserine were analyzed using HPLC, and the analyzed concentrations are shown in Table 10.

### O-Acetylhomoserine Production Medium (pH 7.2)

30 g of glucose, 2 g of KH₂PO₄, 3 g of urea, 40 g of (NH₄)₂SO₄, 2.5 g of peptone, 5 g (10 mL) of corn steep liquor (CSL, Sigma), 0.5 g of MgSO₄·7H₂O, 400 mg of methionine, and 20 g of CaCO₃ (based on 1 L of distilled water)

**Table 10**

| Strains | O-AH (g/L) |
|---|---|
| ATCC13032 ΔmetB ΔmetY lysC (L377K) | 0.7 |
| ATCC13032 ΔmetB ΔmetY lysC (L377K) /pECCG117-metX WT | 1.3 |

As a result, it was confirmed that O-acetyl-L-homoserine was accumulated with a concentration of 0.7 g/L when the ATCC13032 ΔmetB ΔmetY lysC (L377K), as a control, was cultured and O-acetyl-L-homoserine was accumulated with a concentration 1.3 g/L when the ATCC13032 ΔmetB ΔmetY lysC (L377K)/pECCG117-metX WT strain was cultured as shown in Table 10.

Therefore, an increase in production of O-acetyl-L-homoserine was confirmed in the ATCC13032 ΔmetB ΔmetY lysC (L377K)/pECCG117-metX WT strain based on the results shown above.

### 3-6. Evaluation of Ability to Produce Homoserine

*Corynebacterium glutamicum* ATCC13032 ΔmetB ΔmetY lysC (L377K) constructed in Example 3-3 was introduced with the pECCG117-metX WT vector constructed in Example 3-4 using an electric-pulse method and smeared on a selective medium containing kanamycin (25 mg/L) and cultured to obtain transformants.

For comparison of the homoserine producing ability of the strains, the strains were cultured by way of the following method, and homoserine contained in the cultures was analyzed.

One inoculation loop of each of the strains was inoculated to a 250 mL corner-baffled flask containing 25 mL of a medium below and then cultured while shaking at 37°C for 20 hours at 200 rpm. The concentrations of homoserine were analyzed using HPLC, and the analyzed concentrations are shown in Table 11.

### Homoserine Production Medium (pH 7.2)

30 g of glucose, 2 g of KH₂PO₄, 3 g of urea, 40 g of (NH₄)₂SO₄, 2.5 g of peptone, 5 g (10 mL) of corn steep liquor (CSL, Sigma), 0.5 g of MgSO₄·7H₂O, 400 mg of methionine, and 20 g of CaCO₃ (based on 1 L of distilled water)

**Table 11**

| Strains | Homoserine production (g/L) |
|---|---|
| ATCC13032 ΔmetB ΔmetY lysC (L377K) | 0.2 |
| ATCC13032 ΔmetB ΔmetY lysC (L377K)/pECCG117-metX WT | 0.2 |

As a result, it was confirmed that homoserine was accumulated with a concentration of 0.2 g/L when the ATCC13032 ΔmetB ΔmetY lysC (L377K), as a control, was cultured and homoserine was accumulated with a concentration of 0.2 g/L when the ATCC13032 ΔmetB ΔmetY lysC (L377K)/pECCG117-metX WT strain was cultured as shown in Table 11.

Therefore, based on the results, it was confirmed the ATCC13032 ΔmetB ΔmetY lysC (L377K)/pECCG117-metX WT strain had the same level of homoserine production.

### Example 4: Construction of Strain Introduced with Variant Exogenous Membrane Protein (YieH) Having Enhanced Ability to Produce O-Acetylhomoserine and Evaluation of Ability to Produce O-Acetylhomoserine

### 4-1. Introduction of Variant Exogenous Membrane Protein (YjeH)

*Corynebacterium glutamicum* ATCC13032 ΔmetB ΔmetY lysC (L377K) strain constructed in Example 3-3 was transformed with the pDCM2-ΔNCgl2335, pDCM2-ΔNCgl2335::PCJ7-yjeH(eco, WT), pDCM2-ΔNCgl2335::PCJ7-yjeH (eco, F92N), and pDCM2-ΔNCgl2335::PCJ7-yjeH (eco, F351L) vectors previously constructed in Example 2-1 and subjected to a secondary crossover process to obtain ATCC13032 ΔmetB ΔmetY lysC (L377K) ΔNCgl2335, ATCC13032 ΔmetB ΔmetY lysC (L377K) ΔNCgl2335::PCJ7-yjeH (eco, WT), ATCC13032 ΔmetB ΔmetY lysC (L377K) ΔNCgl2335::PCJ7-yjeH(eco, F92N), and ATCC13032 ΔmetB ΔmetY lysC (L377K) ΔNCgl2335::PCJ7-yjeH (eco, F351L) from which the NCgl2335 gene (transposase) was deleted from the chromosome. Insertion of inactivated NCgl2335 gene and newly introduced *E. coli yjeH* gene was identified by performing PCR using the primers of SEQ ID NOS: 4 and 7 and comparing results with those of ATCC13032 in which the NCgl2335 gene was not inactivated.

### 4-2. Construction of O-Acetylhomoserine-producing Strain and Evaluation of Ability to Produce O-Acetylhomoserine

After the pECCG117-metX WT vector prepared in Example 3-4 was introduced into ATCC13032 ΔmetB ΔmetY lysC (L377K) ΔNCgl2335, ATCC13032 ΔmetB ΔmetY lysC (L377K) ΔNCgl2335::PCJ7-yjeH (eco, WT), ATCC13032 ΔmetB ΔmetY lysC (L377K) ΔNCgl2335::PCJ7-yjeH(eco, F92N), and ATCC13032 ΔmetB ΔmetY lysC (L377K) ΔNCgl2335::PCJ7-yjeH (eco, F351L) prepared in Example 4-1 using an electric-pulse method, the strains were smeared on a selective medium containing kanamycin (25 mg/L) and cultured to obtain transformants.

For comparison of the O-acetylhomoserine producing ability of the strains, the strains were cultured by way of the following method, and O-acetylhomoserine contained in the cultures was analyzed.

One inoculation loop of each of the strains was inoculated to a 250 mL corner-baffled flask containing 25 mL of the O-acetylhomoserine production medium (pH 7.2) of Example 3-5 and then cultured while shaking at 33°C for 20 hours at 200 rpm. The concentrations of O-acetylhomoserine were analyzed using HPLC, and the analyzed concentrations are shown in Table 12.

**Table 12**

| Strains | | O-AH (g/L) |
|---|---|---|
| ATCC13032 ΔmetB ΔmetY lysC (L377K) /pECCG117-metX WT | - | 1.3 |
| | ΔNCgl2335 | 1.3 |
| | ΔNCgl2335::PCJ7-yjeH (eco, WT) | 2.1 |
| | ΔNCgl2335::PCJ7-yjeH (eco, F92N) | 2.7 |
| | ΔNCgl2335::PCJ7-yjeH (eco, F351L) | 2.3 |

As a result, it was confirmed that O-acetyl-L-homoserine was accumulated with a concentration of 1.3 g/L when *Corynebacterium glutamicum* ATCC13032 ΔmetB ΔmetY lysC (L377K), as a control, was cultured and production of O-acetyl-L-homoserine was not affected by deletion of the NCgl2335 gene (transposase) as shown in Table 12. Particularly, it was confirmed that O-acetyl-L-homoserine was accumulated with a concentration of 2.1 g/L when the wild-type *yjeH* gene was expressed and O-acetyl-L-homoserine was accumulated with concentrations of 2.7 g/L and 2.3 g/L when the variant *yjeH* genes were expressed.

Therefore, based on the results shown above, it was confirmed that the wild-type and variant *yjeH* genes of the present disclosure significantly increased production of the amino acid of interest by exporting O-acetyl-L-homoserine from the *Corynebacterium glutamicum* ATCC13032.

### 4-3. Evaluation of Ability to Produce Homoserine

After the pECCG117-metX WT vector prepared in Example 3-4 was introduced into ATCC13032 ΔmetB ΔmetY lysC (L377K) ΔNCgl2335, ATCC13032 ΔmetB ΔmetY lysC (L377K) ΔNCgl2335::PCJ7-yjeH (eco, WT), ATCC13032 ΔmetB ΔmetY lysC (L377K) ΔNCgl2335::PCJ7-yjeH(eco, F92N), and ATCC13032 ΔmetB ΔmetY lysC (L377K) ΔNCgl2335::PCJ7-yjeH (eco, F351L) prepared in Example 4-1 using an electric-pulse method, the strains were smeared on a selective medium containing kanamycin (25 mg/L) and cultured to obtain transformants.

For comparison of the homoserine producing ability of the strains, the strains were cultured by way of the following method, and homoserine contained in the cultures was analyzed.

One inoculation loop of each of the strains was inoculated to a 250 mL corner-baffled flask containing 25 mL of a medium below and then cultured while shaking at 33°C for 20 hours at 200 rpm. The concentrations of homoserine were analyzed using HPLC, and the analyzed concentrations are shown in Table 13.

### Homoserine Production Medium (pH 7.2)

30 g of glucose, 2 g of KH₂PO₄, 3 g of urea, 40 g of (NH₄)₂SO₄, 2.5 g of peptone, 5 g (10 mL) of corn steep liquor (CSL, Sigma), 0.5 g of MgSO₄·7H₂O, 400 mg of methionine, and 20 g of CaCO₃ (based on 1 L of distilled water)

**Table 13**

| Strains | | Homoserine (g/L) |
|---|---|---|
| ATCC13032 ΔmetB ΔmetY lysC (L377K) /pECCG117-metX WT | - | 0.2 |
| | ΔNCgl2335 | 0.2 |
| | ΔNCgl2335::PCJ7-yjeH (eco, WT) | 0.2 |
| | ΔNCgl2335::PCJ7-yjeH (eco, F92N) | 1.2 |
| | ΔNCgl2335::PCJ7-yjeH (eco, F351L) | 0.7 |

As a result, it was confirmed that homoserine was accumulated with a concentration of 0.2 g/L when *Corynebacterium glutamicum* ATCC13032 ΔmetB ΔmetY lysC (L377K), as a control, was cultured, and production of homoserine was not affected by deletion of the NCgl2335 gene (transposase) as shown in Table 13. Particularly, it was confirmed that homoserine was accumulated with a concentration of 0.2 g/L when the wild-type *yjeH* gene was expressed, and homoserine was accumulated with concentrations of 1.2 g/L and 0.7 g/L when the variant *yjeH* genes were expressed.

Therefore, based on the results shown above, it was confirmed that the wild-type and variant *yjeH* genes of the present disclosure significantly increased production of the amino acid of interest by exporting homoserine from the *Corynebacterium glutamicum* ATCC13032.

### Example 5: Production of Methionine (MET) Using Conversion Reaction of O-Acetylhomoserine Produced by Strain

After the pECCG117-metX WT vector prepared in Example 3-4 was introduced into ATCC13032 ΔmetB ΔmetY lysC (L377K) ΔNCgl2335, ATCC13032 ΔmetB ΔmetY lysC (L377K) ΔNCgl2335::PCJ7-yjeH (eco, WT), ATCC13032 ΔmetB ΔmetY lysC (L377K) ΔNCgl2335::PCJ7-yjeH(eco, F92N), and ATCC13032 ΔmetB ΔmetY lysC (L377K) ΔNCgl2335::PCJ7-yjeH (eco, F351L) prepared in Example 4-1 using an electric-pulse method, the strains were smeared on a selective medium containing kanamycin (25 mg/L) and cultured to obtain transformants.

Also, according to a method disclosed in a previously filed patent document (WO2008/013432), *metZ* gene encoding O-succinylhomoserine sulfhydrylase derived from *Pseudomonas aeruginosa* and *Pseudomonas putida,* which would be used for conversion of O-acetylhomoserine into methionine, was cloned. PCR was performed by using the chromosome of each strain as a template and primers to obtain the *metZ* gene of *Pseudomonas aeruginosa* and *Pseudomonas putida* as follows: 30 cycles of denaturation at 94°C for 30 seconds, annealing at 55°C for 30 seconds, extension at 72°C for 2 minutes to obtain DNA fragments. The obtained DNA fragments were digested with Ndel/Pacl and cloned into pCL-CJ1 vector (CJ, Korea) digested with the same enzymes. *E. coli* W3110 cells were transformed with the cloned vector and cultured in an LB plate medium containing 50 µg/L spectinomycin, and then colonies were selected. The selected colonies were inoculated onto 3 mL of an LB medium containing 50 µg/L spectinomycin and cultured overnight at 37°C. The cultured cells were recovered, washed with a 0.1 M potassium phosphate buffer (pH 7.5), suspended in 200 µL of the potassium phosphate buffer, and lysed by sonication 5 times at 30 seconds intervals. The cell lysate was centrifuged at 12,000 rpm for 10 minutes and the supernatant was obtained to quantify the total protein level by using a Bio-Rad protein quantification solution (Bio-Rad, USA). Protein expression was identified by SDS-PAGE. The supernatant obtained from the cell extract was used for enzymatic conversion reaction.

Also, *metZ* gene encoding O-succinylhomoserine sulfhydrylase derived from *Hyphomonas neptunium* was cloned. PCR was performed using the chromosome of *Hyphomonas neptunium* as a template and a primer in the same manner as described above to obtain DNA fragments. The obtained DNA fragments were digested with Ndel/Avrll and cloned into pCL-CJ1 vector (CJ, Korea) digested with the same enzymes. A supernatant was obtained using the cloned vector was used to in the same manner as described above and used for enzymatic conversion reaction.

Each of the W3110 strains transformed with the *metZ* expression vector derived from *Pseudomonas* and transformed with the *metZ* expression vector derived from *Hyphomonas* were inoculated onto an LB plate medium containing spectinomycin, as an antibiotic, and cultured overnight at a temperature of 30°C to 40°C. Then, a single colony was inoculated onto 40 mL of the LB medium containing spectinomycin and cultured for 5 hours at a temperature of 30°C to 40°C. The cultured *Pseudomonas*-derived *metZ*-expressing strain and *Hypomononas-*derived *metZ*-expressing were cultured in a 1 L fermentation bath at 600 rpm to 900 rpm, at a temperature of 30°C to 40°C, for 15 to 30 hours to obtain fermentation broths. The culture medium used in the culturing is as follows. The cells of the obtained fermentation broth were lysed by sonication to prepare enzyme solutions.

### 2XYT Medium

10 g of yeast extract, 16 g of tryptophan, 40 g of glucose, and 50 g of spectinomycin (based on 1 L of distilled water)

Methionine conversion reaction was performed by adding the *Pseudomonas-*derived and *Hyphomonas*-derived O-succinylhomoserine sulfhydrylase enzyme solution to the culture of the transformant producing O-acetylhomoserine. After adding 0.1 L of the enzyme solution was added to 2.0 L of the culture of O-acetylhomoserine from which cells were not removed, and 0.3 L of a 15% sodium methyl mercaptan solution was added thereto to initiate reaction. After 2 hours, the fermentation broth was recovered, cells were removed therefrom, and concentrations of the produced methionine were identified by HPLC. The analyzed concentrations are as shown in Table 14.

**Table 14**

| Strains | | Met (g/L) |
|---|---|---|
| ATCC13032 ΔmetB ΔmetY lysC (L377K) /pECCG117-metX WT | - | 1.2 |
| | ΔNCgl2335 | 1.2 |
| | ΔNCgl2335::PCJ7-yjeH (eco, WT) | 1.9 |
| | ΔNCgl2335::PCJ7-yjeH (eco, F92N) | 2.5 |
| | ΔNCgl2335::PCJ7-yjeH (eco, F351L) | 2.1 |

The above description of the present invention is provided for the purpose of illustration, and it would be understood by those skilled in the art that various changes and modifications may be made without changing technical conception and essential features of the present invention. Thus, it is clear that the above-described embodiments are illustrative in all aspects and do not limit the present invention. Therefore, the scope of the disclosure is defined not by the detailed description, but by the claims and their equivalents, and all variations within the scope of the claims and their equivalents are to be construed as being included in the disclosure.

## Claims

1. A variant of an inner membrane protein YjeH comprising i) substitution of an amino acid corresponding to the 92^{nd} position with asparagine, ii) substitution of an amino acid corresponding to the 351^{st} position with leucine, or iii) substitution of both i) and ii), in an amino acid sequence of SEQ ID NO: 1, and having a homology of 95% or more and less than 100% with SEQ ID NO: 1.

2. The variant according to claim 1, wherein the variant has an ability to export O-acetylhomoserine or homoserine.

3. A polynucleotide encoding the variant of claim 1.

4. A vector comprising the polynucleotide of claim 3.

5. A microorganism comprising at least any one of: a variant of an inner membrane protein YjeH comprising i) substitution of an amino acid corresponding to the 92^{nd} position with asparagine, ii) substitution of an amino acid corresponding to the 351^{st} position with leucine, or iii) substitution of both i) and ii), in an amino acid sequence of SEQ ID NO: 1, and having a homology of 95% or more and less than 100% with SEQ ID NO: 1; and a polynucleotide encoding the variant.

6. The microorganism according to claim 5, wherein the microorganism has an enhanced ability to produce O-acetylhomoserine or homoserine compared to non-modified microorganism.

7. The microorganism according to claim 5, wherein the microorganism belongs to the genus *Corynebacterium.*

8. A method for producing a target product, the method comprising culturing a microorganism in a culture medium, wherein the microorganism comprises at least any one of: a variant of an inner membrane protein YjeH comprising i) substitution of an amino acid corresponding to the 92^{nd} position with asparagine, ii) substitution of an amino acid corresponding to the 351^{st} position with leucine, or iii) substitution of both i) and ii), in an amino acid sequence of SEQ ID NO: 1, and having a homology of 95% or more and less than 100% with SEQ ID NO: 1; and a polynucleotide encoding the variant.

9. The method according to claim 8, the method further comprising recovering a target product from the culture medium or the microorganism.

10. The method according to claim 8, wherein the target product is *O-*acetylhomoserine or homoserine.

11. A method for producing methionine, the method comprising:
producing O-acetylhomoserine by culturing a microorganism in a culture medium, the microorganism comprising at least any one of: a variant of an inner membrane protein YjeH comprising i) substitution of an amino acid corresponding to the 92^{nd} position with asparagine, ii) substitution of an amino acid corresponding to the 351^{st} position with leucine, or iii) substitution of both i) and ii), in an amino acid sequence of SEQ ID NO: 1, and having a homology of 95% or more and less than 100% with SEQ ID NO: 1; and a polynucleotide encoding the variant; and
converting the O-acetylhomoserine into methionine by reacting the O-acetylhomoserine with a sulfide.

12. A method for producing glufosinate, the method comprising:
producing O-acetylhomoserine or homoserine by culturing a microorganism in a culture medium, the microorganism comprising at least any one of: a variant of an inner membrane protein YjeH comprising i) substitution of an amino acid corresponding to the 92^{nd} position with asparagine, ii) substitution of an amino acid corresponding to the 351^{st} position with leucine, or iii) substitution of both i) and ii), in an amino acid sequence of SEQ ID NO: 1, and having a homology of 95% or more and less than 100% with SEQ ID NO: 1; and a polynucleotide encoding the variant; and
converting the O-acetylhomoserine or homoserine into glufosinate.

13. A composition for producing homoserine comprising the microorganism of claim 5 or a culture of the microorganism.

14. A composition for producing O-acetylhomoserine comprising the microorganism of claim 5 or a culture of the microorganism.

15. A use of the variant of the inner membrane protein YjeH of claim 1 for producing O-acetylhomoserine or homoserine.

16. A use of the microorganism of claim 5 for producing O-acetylhomoserine or homoserine.
